(19) Europäisches Patentamt

European Patent Office

Office européen des brevets

(11) **EP 4 710 841 A1**

(12) **EUROPEAN PATENT APPLICATION**

(43) Date of publication:
**18.03.2026 Bulletin 2026/12**

(21) Application number: **24200913.2**

(22) Date of filing: **17.09.2024**

(51) International Patent Classification (IPC):
**A61B 5/00** (2006.01)     **A61B 3/10** (2006.01)
**G01B 9/02055** (2022.01)     **G01B 9/02091** (2022.01)

(52) Cooperative Patent Classification (CPC):
**A61B 3/102; A61B 3/18; A61B 5/0066;
G01B 9/0205; G01B 9/02063; G01B 9/02091**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Carl Zeiss Meditec AG
07745 Jena (DE)**

(72) Inventors:
- **MacDougall, Daniel
  Halifax, B3H0A8 (CA)**
- **Adamson, Robert
  Halifax, B3H0A8 (CA)**
- **Rybakovski, Oleg
  220076 Minsk (BY)**

(74) Representative: **Carl Zeiss AG - Patentabteilung
Carl-Zeiss-Straße 22
73447 Oberkochen (DE)**

(54) **HANDHELD OPTICAL COHERENCE TOMOGRAPHY (OCT) SYSTEM WITH INTEGRATED IMAGING CAMERA FOR COMBINED OCT IMAGING AND CONVENTIONAL IMAGING**

(57) An optical system comprising a distal lens assembly defining an optical exit axis; an optical beam combining element; an image sensor; an imaging lens assembly defining an imaging beam path between the optical beam combining element and the image sensor, the distal lens assembly and the imaging lens assembly configured such that imaging light scattered by a sample residing at a distal object plane is collected by the distal lens assembly and directed onto the image sensor by the imaging lens assembly according to an optical imaging beam path; an optical coherence tomography beam delivery assembly configured to deliver, from an optical coherence tomography subsystem, an optical coherence tomography sample arm beam onto the optical beam combining element such that the optical coherence tomography sample arm beam is directed through the distal lens assembly onto the sample, thereby defining an optical coherence tomography sample arm beam path, and such that reflected optical coherence tomography light is collected by the distal lens assembly and directed to the optical coherence tomography subsystem for detection, wherein the distal lens assembly and/or the imaging lens assembly and/or the optical coherence tomography beam delivery assembly comprises a variable focal length lens configured to variably adjust a distance between the distal lens assembly and the distal object plane along the optical exit axis, wherein a focal length of the variable focal length lens is variable within a predefined focal length range.

FIG. 4A

## Description

### TECHNICAL FIELD

The present disclosure relates to handheld diagnostic devices and methods of use thereof. In some aspects, the present disclosure relates to devices and methods for vibrometric assessment and imaging of the ear.

### SUMMARY

There may be a desire for an improved optical system which is able to visualize structures hidden by the tympanic membrane, i.e. the ossicular chain.

The subject-matter of the independent claims solves the above-mentioned problems. Advantages embodiments of the invention are subject matter of the dependent claims.

In one embodiment of the present disclosure, the optical system comprises a distal lens assembly. The distal lens assembly can include one or more than one lens, i.e. two, three, four, five, six, or seven lenses. It can also include more than seven lenses. The distal lens assembly defines an optical exit axis. The optical exit axis is the axis around which the exit of the optical system is defined. The exit of the optical system is defined by the part of the optical system which approaches the tympanic membrane when the optical system is inserted into an ear.

The optical system further comprises an optical beam combining element. The optical beam combining element can be a beamsplitter. A beamsplitter is an optical component used to split incident light at a designated ratio into two separate beams. However, a beamsplitter can be used in reverse to combine two different beams into a single one. The beamsplitter can be a dichroic mirror.

The optical system further comprises an image sensor. The image sensor can be capable of capturing the light which traveled from the ear through the optical system to the image sensor. The image sensor can be a CCD sensor, a CMOS sensor, a EMCCD sensor, a SCMOS sensor, photo diode arrays or something similar.

The optical system further comprises an imaging lens assembly. The imaging lens assembly can include one or more than one lens, i.e. two, three, four, five, six, or seven lenses. It can also include more than seven lenses. The imaging lens assembly defines an imaging beam path between the optical beam combining element and the image sensor.

The distal lens assembly and the imaging lens assembly are configured such that imaging light scattered by a sample residing at a distal object plane is collected by the distal lens assembly and directed onto the image sensor by the imaging lens assembly according to an optical imaging beam path. The sample can be a middle-ear structure, e.g. the tympanic membrane.

The optical system further comprises an optical coherence tomography beam delivery assembly. The optical coherence tomography beam delivery assembly is configured to deliver, from an optical coherence tomography subsystem, an optical coherence tomography sample arm beam onto the optical beam combining element such that the optical coherence tomography sample arm beam is directed through the distal lens assembly onto the sample, thereby defining an optical coherence tomography sample arm beam path, and such that reflected optical coherence tomography light is collected by the distal lens assembly and directed to the optical coherence tomography subsystem for detection.

The distal lens assembly and/or the imaging lens assembly and/or the optical coherence tomography beam delivery assembly comprises a variable focal length lens configured to variably adjust a distance between the distal lens assembly and the distal object plane along the optical exit axis, wherein a focal length of the variable focal length lens is variable within a predefined focal length range.

Preferably, the variable focal length lens is arranged within the imaging lens assembly.

Preferably, the imaging lens assembly additionally comprises a first lens or first lens group with positive refractive power. Preferably, the imaging lens assembly additionally comprises a second lens or second lens group with positive refractive power. A lens with a positive refractive power can be a converging lens. i.e. a convex lens. Preferably, the first lens or the first lens group is positioned between the optical beam combining element and the second lens or second lens group. Preferably, a limiting aperture is arranged between the first lens or first lens group and the second lens or second lens group. The limiting aperture can be an optical aperture, e.g. a pinhole.

Preferably, the variable focal length lens is positioned in the proximity to the limiting aperture.

Preferably, the variable focal length lens is positioned adjacent to the limiting aperture.

Preferably, the first lens or first lens group and the distal lens assembly are configured to generate an image of the limiting aperture in proximity to a distal end of the distal lens assembly, the image of the limiting aperture thereby defining an entrance pupil of an optical imaging beam path leading from the distal object plane to the image sensor.

Preferably, the optical coherence tomography beam delivery assembly comprises a scanning mirror and one or more than one additional lens configured to conjointly with the distal lens assembly generate an image of the scanning mirror, or more precisely a scanning axis of the scanning mirror, in proximity to the distal end of the distal lens assembly. Preferably, the image of the scanning mirror, or the position of the image of the scanning axis of the scanning mirror, defines an exit pupil of the optical coherence tomography sample arm beam.

Preferably, the first lens or first lens group, the one or more additional lenses, the distal lens assembly and a positioning of the variable focal length lens are configured such that a distance between an entrance pupil associated with an optical imaging beam path leading from the distal object plane to the image sensor and the exit pupil associated with the optical coherence tomography sample arm beam path remains less than 4 mm when the variable focal length lens is varied throughout the predefined focal length range.

Preferably, the first lens or lens group, the one of more additional lenses, the distal lens assembly and a positioning of the variable focal length lens are configured such that a mis-registration between an image recorded with the image sensor of objects laying along a same OCT path remains smaller than 1 mm when the variable focal length lens is varied throughout the predefined focal length range.

Preferably, the limiting aperture is defined by a pinhole.

Preferably, the optical beam combining element is a dichroic mirror.

Preferably, the variable focal length lens is a liquid lens.

Preferably, the optical coherence tomography subsystem is configured such that a change in an axial location of the distal object plane caused by actuation of the variable focal length lens is accompanied with a corresponding change in an axial location of an imaging depth region of the optical coherence tomography sample arm beam.

Preferably, the axial location of the axial focal region of the optical coherence tomography sample arm beam is varied by changing a length of a reference arm of the optical coherence tomography subsystem. A change adjustment in the length of the reference arm of the optical coherence tomography subsystem can be approximately one half a change in a distance of the object plane from the exit of the optical system achieved through adjustment of the focal length of the variable focal length lens.

Preferably, the optical system is integrated into a handheld device.

In another embodiment of the present disclosure, the optical system comprises a distal lens assembly. The distal lens assembly can include one or more than one lens, i.e. two, three, four, five, six, or seven lenses. It can also include more than seven lenses. The distal lens assembly defines an optical exit axis. The optical exit axis is the axis around which the exit of the optical system is defined. The exit of the optical system is defined by the part of the optical system which approaches the tympanic membrane when the optical system is inserted into an ear.

The optical system further comprises an optical beam combining element. The optical beam combining element can be a beamsplitter. A beamsplitter is an optical component used to split incident light at a designated ratio into two separate beams. However, a beamsplitter can be used in reverse to combine two different beams into a single one. The beamsplitter can be a dichroic mirror. The optical system further comprises an image sensor. The image sensor can be capable of capturing the light which traveled from the ear through the optical system to the image sensor. The image sensor can be a CCD sensor, a CMOS sensor, a EMCCD sensor, a SCMOS sensor, photo diode arrays or something similar.

The optical system further comprises an imaging lens assembly. The imaging lens assembly can include one or more than one lens, i.e. two, three, four, five, six, or seven lenses. It can also include more than seven lenses. The imaging lens assembly defines an imaging beam path between the optical beam combining element and the image sensor.

The distal lens assembly and the imaging lens assembly are configured such that imaging light scattered by a sample residing at a distal object plane is collected by the distal lens assembly and directed onto the image sensor by the imaging lens assembly according to an optical imaging beam path. The sample can be a middle-ear structure, e.g. the tympanic membrane.

The optical system further comprises an optical coherence tomography beam delivery assembly. The optical coherence tomography beam delivery assembly is configured to deliver, from an optical coherence tomography subsystem, an optical coherence tomography sample arm beam onto the optical beam combining element such that the optical coherence tomography sample arm beam is directed through the distal lens assembly onto the sample, thereby defining an optical coherence tomography sample arm beam path, and such that reflected optical coherence tomography light is collected by the distal lens assembly and directed to the optical coherence tomography subsystem for detection.

The optical system further comprises an optical filter, located within the imaging beam path, wherein the optical filter is configured to attenuate scattered laser light of the optical coherence tomography subsystem. The optical filter may be a light-blocking filter, e.g. a spectral edge filter or a bandpass filter being non-transmissive for the wavelength of the OCT light and transmissive for the light scattered by a sample residing at a distal object plane and imaged by the image sensor. This filter may serve to prevent even small amounts of scattered OCT light transmitted through the optical beam combining element. The optical filter improves the simultaneous recording and displaying of the middle ear structures on a user interface, during acquisition of the OCT data. Such "leakage" of OCT light detected by the imaging sensor otherwise may result in artifacts on the middle-ear structure image, hampering the interpretation of such images and potentially leading to problems in understanding the actual position of the OCT beam when the middle-ear structure image is annotated with OCT beam scanning annotations.

Preferably, the optical system is integrated into a handheld device.

In another embodiment of the present disclosure, a system comprises an optical system according to one of the above-mentioned embodiments, a control, and a control interface. The control is configured to adjust a length of a reference arm of the optical coherence tomography subsystem and a focal length of a variable focal length lens due to a user input to the control interface.

Preferably, the optical coherence tomography subsystem is an optical coherence tomography vibrometry subsystem. Preferably, the optical coherence tomography vibrometry subsystem comprises a speaker and a microphone integrated into a handpiece.

Preferably, the optical coherence tomography subsystem is one of a swept source optical coherence tomography subsystem and a spectral domain optical coherence tomography subsystem. Hence, the optical coherence tomography subsystem can be a swept source optical coherence tomography subsystem or a spectral domain optical coherence tomography subsystem.

In another embodiment of the present disclosure, a handheld optical device comprises a housing. The housing comprises a body portion and a handle portion extending from the body portion.

The handheld optical device further comprises an optical system.

The optical system comprises a distal lens assembly. The distal lens assembly can include one or more than one lens, i.e. two, three, four, five, six, or seven lenses. It can also include more than seven lenses. The distal lens assembly defines an optical exit axis. The optical exit axis is the axis around which the exit of the optical system is defined. The exit of the optical system is defined by the part of the optical system which approaches the tympanic membrane when the optical system is inserted into an ear.

The optical system further comprises an optical beam combining element. The optical beam combining element can be a beamsplitter. A beamsplitter is an optical component used to split incident light at a designated ratio into two separate beams. However, a beamsplitter can be used in reverse to combine two different beams into a single one. The beamsplitter can be a dichroic mirror.

The optical system further comprises an image sensor. The image sensor can be capable of capturing the light which traveled from the ear through the optical system to the image sensor. The image sensor can be a CCD sensor, a CMOS sensor, a EMCCD sensor, a SCMOS sensor, photo diode arrays or something similar.

The optical system further comprises an imaging lens assembly. The image lens assembly can include one or more than one lens, i.e. two, three, four, five, six, or seven lenses. It can also include more than seven lenses. The image lens assembly defines an imaging beam path between the optical beam combining element and the image sensor.

The distal lens assembly and the imaging lens assembly are configured such that imaging light scattered by a sample residing at a distal object plane is collected by the distal lens assembly and directed onto the image sensor by the imaging lens assembly according to an optical imaging beam path. The sample can be a middle-ear structure, e.g. the tympanic membrane.

The optical system further comprises an optical coherence tomography beam delivery assembly. The optical coherence tomography beam delivery assembly is configured to deliver, from an optical coherence tomography subsystem, an optical coherence tomography sample arm beam onto the optical beam combining element such that the optical coherence tomography sample arm beam is directed through the distal lens assembly onto the sample, thereby defining an optical coherence tomography sample arm beam path, and such that reflected optical coherence tomography light is collected by the distal lens assembly and directed to the optical coherence tomography subsystem for detection.

The distal lens assembly and the optical beam combining element reside within the body portion.

At least one of the optical imaging lens assembly and the optical coherence tomography beam delivery assembly resides at least partly in the handle portion, i.e. the optical imaging lens assembly and/or the optical coherence tomography beam delivery assembly resides at least partly, i.e. partly or completely, in the handle portion.

Preferably, the optical coherence tomography beam delivery assembly comprises a folding mirror in the beam path between an end face of an optical fiber and a scanning mirror.

Preferably, the handle portion extends in a direction including an oblique angle relative to the optical exit axis in a range between 95 degrees and 120 degrees. A further understanding of the functional and advantageous aspects of the disclosure may be realized by reference to the following detailed description and drawings.

**BRIEF DESCRIPTION OF THE FIGURES**

The accompanying drawings are incorporated herein and form a part of the specification.

FIG. 1A illustrates a swept-source optical coherence tomography (SS-OCT) system, according to some embodiments.

FIG. 1B illustrates a spectral-domain optical coherence tomography (SD-OCT) system, according to some embodiments.

FIG. 1C depicts a system for performing Doppler OCT vibrometry, according to some embodiments.

FIG. 2 depicts the spatial and temporal optical phase data, extracted from a set of interferograms acquired during the application of an acoustic stimulus using a Doppler OCT vibrometry system, according to some embodiments.

FIG. 3 illustrates a block diagram of an ear imaging system, according to some embodiments.

FIG. 4A depicts an illustration of the sectional view of an optical system of a handheld OCT beam delivery and imaging device, according to some embodiments.

FIG. 4B depicts a distal lens assembly of an optical system of a handheld OCT beam delivery and imaging device, according to some embodiments.

FIG. 4C depicts a proximal lens assembly of an optical system of a handheld OCT beam delivery and imaging device, according to some embodiments.

FIG. 5A depicts an illustration of the sectional view of optical system of a handheld OCT beam delivery and imaging device, according to some embodiments.

FIG. 5B depicts a distal lens assembly of an optical system of a handheld OCT beam delivery and imaging device, according to some embodiments.

FIG. 5C depicts a proximal lens assembly of an optical system of a handheld OCT beam delivery and imaging device, according to some embodiments.

FIG. 6 illustrates a block diagram of a handheld OCT beam delivery and imaging device according to some embodiments.

FIG. 7 illustrates a control and processing circuitry for controlling a handheld OCT beam delivery and ear imaging device and associated OCT subsystem according to some embodiments.

FIG. 8A illustrates a window on a computer display for an ear imaging system in brightness mode (B-mode), with the diagonal line across the infrared image illustrating the slice selected for showing in B-mode and the upper and lower horizontal lines across the B-mode image illustrating the depth selected, according to some embodiments.

FIG. 8B illustrates a window on a computer display for Doppler imaging mode, with left and right vertical lines containing a region with vibration displacement amplitude information in false color, according to some embodiments.

FIG. 9 illustrates an optical system for combining an OCT beam and an en face image in which there is an axial offset between the entrance pupil of the image beam path and the image of the axis of the OCT scanning mirror in object space, according to some embodiments.

FIG. 10 illustrates an optical system for combining an OCT beam with an en face image in which the limiting aperture is located between a variable focal lens and the image plane and in which the principal object plane of the lens does not coincide with the limiting aperture of the imaging system, according to some embodiments.

FIG. 11 illustrates an optical system for combining an OCT beam with an en face image in which a variable focal length lens is located between a limiting aperture and an image plane and in which the principal image plane of the lens does not coincide with the limiting aperture of the imaging system, according to some embodiments.

FIG. 12 illustrates an optical system for combining an OCT beam with an en face image in which the plane of a variable focal length lens is coincident with the plane of the limiting aperture and in which the image of the OCT scanning mirror axis is coincident with the entrance pupil of the imaging system, according to some embodiments.

FIG. 13 illustrates a control and processing system for controlling operation of the OCT subsystem and the handheld OCT beam delivery and imaging device, according to some embodiments.

In the drawings, like reference numbers generally indicate identical or similar elements. Additionally, generally, the left-most digit(s) of a reference number identifies the drawing in which the reference number first appears.

## DETAILED DESCRIPTION

Conductive hearing loss has a 7% prevalence in the adult population and affects roughly 420M people worldwide. Conductive hearing loss is typically caused by a failure of the middle ear - the tympanic membrane and ossicular chain - to effectively conduct sound from the ear canal to the cochlea. This failure in middle ear function may be due to fixation, caused by, for example, the ingrowth of soft tissues or adhesions, stiffening of ossicular joints, the formation of plaque on the stapes footplate, congenital malformation etc. or to discontinuity in the ossicular chain due, for example, to erosion of the ossicles, traumatic injury or congenital defects. Conductive hearing loss may also be caused by perforation or sclerosis of the tympanic membrane, middle ear fluid or a number of less common reasons.

For many of these disorders, surgical intervention may be undertaken to restore hearing. Interventions include, for instance, inserting drainage tubes in the tympanic membrane, replacement of ossicles with prostheses and procedures to close off tympanic membrane perforations. To select the correct intervention and to be able to inform patients about their treatment options, ear specialists have a need to obtain accurate ear diagnoses in the clinic prior to performing surgery.

Ear specialists have a number of diagnostic tools available to them. Audiometry may distinguish conductive hearing loss from the more common sensorineural hearing loss by the presence of an Air-Bone Gap in the audiogram. Tympanometry may identify abnormal eardrum stiffness or compliance that may be indicative, respectively, of a fixation or discontinuity. Computed tomography and magnetic resonance imaging are non-invasive imaging modalities that are sometimes used to image the middle ear. Microscopic examination of the eardrum may help to assess the state of the tympanic membrane and may sometimes provide information about the middle ear, although the opacity of the eardrum prevents the middle ear from being clearly imaged through simple microscopy.

An otoscope is a specialized microscope for visualizing the tympanic membrane that includes a handle, a disposable plastic speculum, an illumination source, and an imaging head containing imaging optics. Recently, video otoscopes have become common, such as those that are described in U.S. Patent Nos. 5,363,839 and 5,919,130. These devices have the form factor of a traditional otoscope but contain a digital camera that may take pictures of the tympanic membrane.

Optical coherence tomography (OCT) is an optical interferometric imaging technology that may produce depth-resolved images of sub-surface tissue structures. This is accomplished by taking a spatially coherent infrared light-source and splitting it between a reference beam and a sample probing beam. Light that is backscattered from structures within the sample are collected and interfered (combined) with the reference beam light in order to produce an interference pattern that, once processed, reveals the location of light-reflecting structures in the sample.

OCT is able to "see" through the tympanic membrane to visualize the structures in the middle ear. In addition, as described below, Doppler OCT (also known as phase-sensitive OCT) may be used to measure the response of ear structures to sound, a feature that may help clinicians in localizing functional defects like fixations and discontinuities, examples of which are described in Pitris, C. et al., "High-resolution imaging of the middle ear with optical coherence tomography: A feasibility study," Arch Otolaryngol Head Neck Surg., 127, pp. 637-642, (2001); U.S. Patent No. 8,115,934; and U.S. Patent Publication No. 2017/0251924.

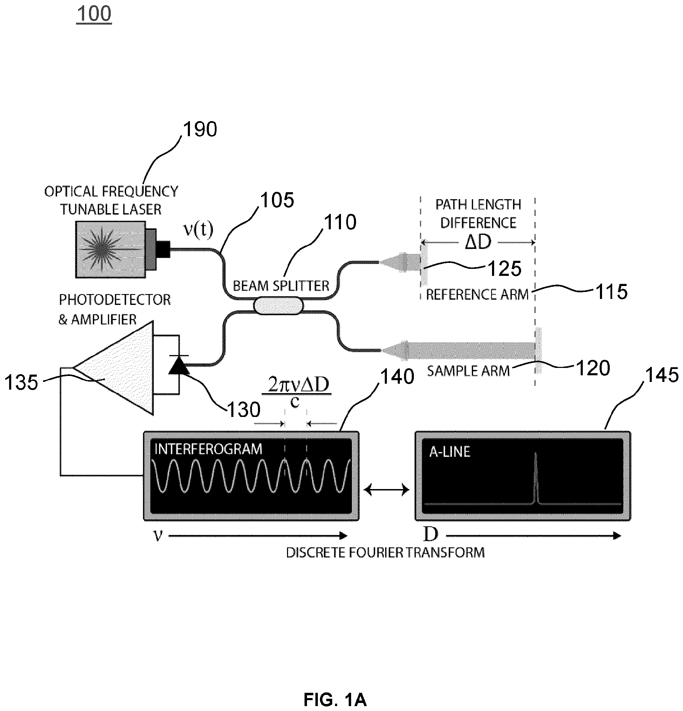

FIG. 1A illustrates a swept-source optical coherence tomography (SS-OCT) system 100, according to some embodiments. OCT measurements may be performed using various approaches, either in the time domain (TD-OCT) or in the frequency domain (e.g., Fourier transform approaches such as spectral domain (SD-OCT) or swept-source (SS-OCT)). The most recent of these to see significant advancements is SS-OCT, as illustrated in FIG. 1A. In SS-OCT, a wavelength-tunable laser 190 is used as the light source to probe the sample. By varying or "sweeping" the optical wavelength of light emanating from the laser, an interference pattern may be detected at many wavelengths and frequency analysis of the detected signal may be used to identify the z-position of a light-reflecting structure in a sample.

In the SS-OCT system 100, at each of the plurality of frequencies, light emanating from the wavelength-tunable laser 190 passes through optical fiber 105 into a beamsplitter 110 that splits the beam into reference 115 and sample 120 arms with a path length difference $\Delta D$. The beam of the reference arm 115 follows optical fiber 105 to its exit and then is reflected by a mirror 125, while the beam of the sample arm 120 follows optical fiber 105 to its exit and then contacts an area on the sample. Structures within the sample area may then reflect some of the beam back to the optical fiber 105. The two beams interfere at the beamsplitter 110, and the intensity of the interfered light is detected at photodetector 130, amplified by amplifier 135, and recorded in a computer (not shown). As the optical frequency, , is swept in time, interference fringes are generated in the measured interferogram 140 with frequencies that are proportional to the path length difference between reflectors I the sample arm and the reference arm. A hypothetical depiction of such an interferogram 140 is shown in the frequency domain (amplitude over time, 140), which is transformed into an A-line 145 (amplitude over distance, bottom right) using discrete Fourier analysis software running on the computer.

The interference fringes are measured by the photodetector 130 (which may be a photodiode) and frequency analysis is performed on the signal to identify the weights of different frequencies, each of which represents a reflector at a different depth in the sample. By sampling of the interference patterns produced by a wavelength sweep profile such that the sampling occurs at evenly spaced optical frequency intervals, or by sweeping the laser linearly in optical frequency and regularly sampling the interference pattern, etc., a reflectivity depth-profile of the sample along the beam path (called an "A-line" or "A-scan" as seen in FIG. 1A) may be obtained by taking the magnitude of the inverse discrete-Fourier-transform (DFT) of the sampled interferograms. 2D (x by z, where z is defined along the axial direction of the beam) brightness mode images (called "B-mode" or "B-scan") may be constructed by scanning the beam across a field-of-view in x and stitching together adjacent A-Lines into an intensity map. Similarly, 3D B-mode volume renders of structures may be constructed from a stacked set of 2D B-mode images collected at various y positions. In medical diagnostics, B-mode images provide structural anatomical information which may be used to distinguish normal structures from pathological ones.

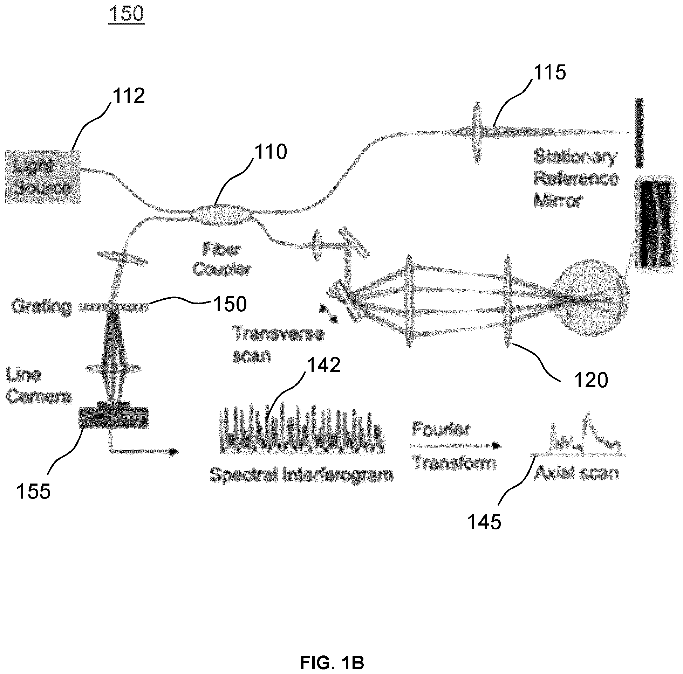

FIG. 1B illustrates a spectral-domain optical coherence tomography (SD-OCT) system 150, according to some embodiments. In the SD-OCT system 150, a beamsplitter 110 splits light from a broadband source 112 between a reference arm 115 and a sample arm 120 and the light reflected from the two arms is interfered at another beamsplitter 110 (which, in some embodiments is just the first beamsplitter used again). The interfered light is dispersed using a dispersive optic 150 such as a dispersion grating, and the spectrum of the signal is recorded using a photodetector array (line camera) 155. The spectrum 142 is the Fourier transform of the axial scan line 145 (A-line) giving the reflectivity of the tissue as a function of depth.

OCT may be applied to imaging the human tympanic membrane and middle ear. It has been shown that anatomical structures within the middle ear may be imaged using OCT; that the tympanic membrane may be imaged in patients using OCT; and that Doppler OCT, described herein, may be used to perform functional imaging in the human middle ear by measuring the vibration of middle ear structures in response to sound. The magnitude of the discrete Fourier transform (DFT) of the interferograms contains structural information about sub-surface reflectors, and the phase of the DFT contains dynamic information. Repeatedly acquired A-lines at the same x, y position of moving objects will contain phase differences that reflect the structures' motion in z. Doppler OCT systems may derive additional image contrast from this phase information and may quantify dynamics. To date, the basic approach that has been taken to extracting magnitude-of-vibration information in non-real-time, benchtop Doppler OCT relies on an acoustic stimulus that is applied to the ear; the acoustic frequency phase variations are then collected over many consecutive complete acoustic cycles and analyzed using Fourier analysis.

For meaningful information to be extracted from the changes in phase of the sampled interferograms, Doppler OCT requires a high degree of phase stability. Doppler OCT is very compatible with SD-OCT as its lack of moving-parts and tuning mechanisms inherently provide very high wavelength repeatability, however Doppler SD-OCT has limited depth scanning range due to the limited spectral resolution of compact and/or high-speed spectrometers, making it less-attractive for use in imaging applications requiring more than a few millimeters of scanning range such as middle ear imaging. An important requirement for interferometric phase stability in SD-OCT and SS-OCT is wavelength-repeatability. Non-limiting examples of suitable systems for performing Doppler OCT are disclosed in International Patent Application No. PCT/CA2016/051199, titled "SYSTEMS AND METHODS FOR SWEPT SOURCE OPTICAL COHERENCE TOMOGRAPHY VIBROGRAPHY" and filed on October 14, 2016, which is incorporated herein by reference in its entirety.

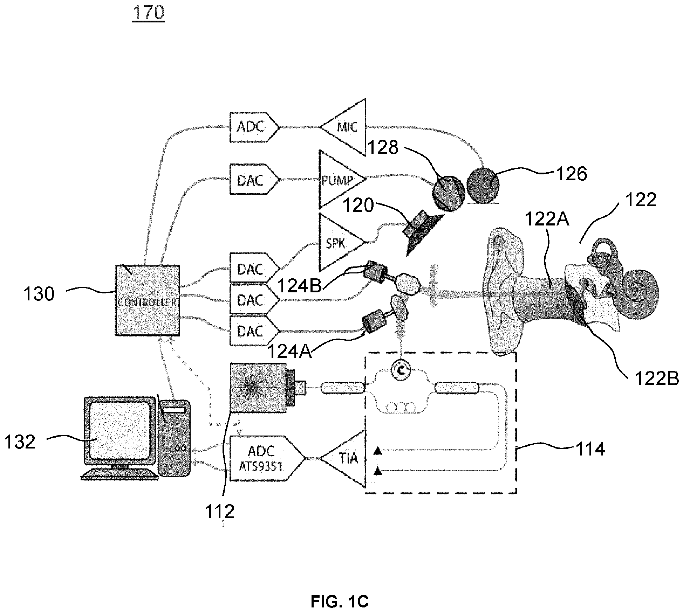

FIG. 1C shows a system 170 for performing Doppler OCT vibrometry, according to some embodiments. A Doppler OCT optical coherence tomography vibrometry system 170 is depicted. This non-limiting example system may include an akinetic swept-source laser 172, an interferometer 114, high speed digitization electronics 116, and a sound delivery system 118 for delivering a sound stimulus to an ear 122 via a speaker 120. Laser light from the interferometer 114 is directed into the ear 22 by scanning mirrors 124A and 124B, and scattered light is collected and directed back to the interferometer 114, this forming the sample arm of the interferometer 114.

The sound stimulus provided by speaker 120 is synchronized to a sweep clock signal of laser 112 in such a way that the acoustic phase of the stimulus is advanced in coordination with each sweep of laser 112. The sweep clock signal is a clock signal generated by laser 112 or used to drive laser 112 such that an edge of the sweep clock signal occurs in a fixed timing relationship to the beginning of the frequency sweep of laser 112. Synchronization of the sweep clock signal and sound stimulus ensures that the measured optical phase in the resulting image of ear 122 will advance at the same rate as the phase of the acoustic stimulus resulting in a stable phase shift between the acoustic stimulus and the measured optical phase at each voxel in the image.

The microphone 126 records the pressure in ear canal 122A in response to the acoustic stimulus generated by speaker 120. A pump 128 may be provided to maintain a desired static pressure in ear canal 122A. The laser 112, speaker 120, scanning mirrors 124A and 124B, microphone 126, and pump 128 may all be connected to a controller 130. Controller 130 processes signals from interferometer 114 to produce an interferogram representing one or more structures and/or encoding vibrometric features of one or more structures within the ear canal 122A and/or eardrum 22B. Scanning mirrors 124A and 124B may scan the beam from laser 112 laterally across the field of view.

While FIG. 1C shows a mirror-based scanning subsystem, in other embodiments, one or more of the mirrors may be replaced by other scanning elements. Non-limiting examples of non-mirror based scanning elements include acousto-optic deflectors, electro-optic deflectors, liquid crystal spatial light modulators, and photorefractive scanners.

It will be understood that the system 170 shown in FIG. 1C is not intended to be limiting, and that other Doppler optical coherence tomography imaging system configurations may be employed in the alternative. For example, in some embodiments, systems and methods described herein may be adapted to spectral domain optical coherence tomography (SD-OCT) vibrometry.

SD-OCT systems direct the collected and interfered light onto a spectrometer and the intensity at discrete optical wavelengths is recorded by, for instance, a CMOS detector array, charge-coupled device (CCD) or photodiode array. In SD-OCT systems using this approach, the detector array signal may be appropriately resampled to obtain measurements that are linearly spaced in optical frequency, and then Fourier transformed forming an A-scan signal. The phase of consecutive A-scans is multiplied by in-phase and quadrature sinusoids and averaged. The embodiments described herein in the context of swept-source OCT vibrometry, may be adapted to SD-OCT systems, for example, by employing the A-scan trigger, employed to trigger the acquisition of a spectrum from the detector array, in the place of the sweep trigger, such that the scanning subsystem and the acoustic stimulus subsystem are synchronized with the A-scan trigger.

As shown in FIG. 1C, the system 170, and other Doppler OCT vibrometry systems, may be employed for ontological vibrometric measurements. When an acoustic tone is presented to the ear, the middle ear structures (the eardrum and ossicles) are set into periodic motion at the acoustic frequency as they conduct sound energy to the inner ear. When the vibrating structures are interrogated via Doppler (phase-sensitive) OCT vibrometry, phase variations in the interferometric signal appear at the acoustic frequency as well. While the audible range of acoustic frequencies is typically cited as 20 Hz - 20kHz, the range of diagnostically useful frequencies is limited to 100 Hz to 10 kHz.

The system in FIG. 1C may be employed to perform a single vibrometric measurement at a selected lateral scanning location, or, for example, to collect vibrometric image data among a plurality of locations. In some embodiments, multi-frame averaging is employed to interleave B-mode imaging with the generation of vibrometric measures, based on the use of the sweep trigger to control and synchronization of the phase evolution of the acoustic stimulus among multiple image frames.

In some embodiments, vibrometric signal processing may be performed according to the following mathematical relations. A given A-scan line (image line) contains Y pixels in the depth domain, indexed by y, and is calculated from a set of acquired interferograms, each one corresponding a laser sweep, as indexed by n. Given the desired acoustic frequency, $f_{acoustic}$, and a laser sweep rate, $f_{sweep}$, the amount of acoustic phase in radians accumulated during any laser sweep, n, of a given image line is constant and given by the expression, where there are N laser sweeps per acoustic cycle:

$$\Delta\phi = 2\pi \frac{f_{acoustic}}{f_{sweep}} = \frac{2\pi}{N}.$$

Therefore, the total amount of acoustic phase accumulated during n laser sweeps is given by $n \times \Delta\phi$. It

is therefore expected that the dependence of the measured optical phase (i.e. the phase of the A-scans, that is to say, the discrete Fourier transform (DFT) of the interferograms) of a vibrating reflector located at pixel y, on the laser sweep number n (effectively a time index) to be described, as follows:

$$\psi_{y,n} = \frac{4\pi A_y}{\lambda_0}\sin(\frac{2\pi n}{N} + \phi_y) + \psi_{y,0} + \psi_{noise},$$

where $A_y$ is the amplitude of acoustic vibration, $\lambda_0$ is the center optical wavelength, $\phi_y$ is the acoustic phase shift of the reflector resulting from its dynamic response characteristics, $\psi_{y,0}$ is the optical phase that would be measured with no acoustic stimulus present (i.e. a stationary sample), and $\psi_{noise}$ is the optical phase noise in the system owing to thermal drifts, mechanical noise, shot noise, and wavelength repeatability in the laser. Accordingly, after performing an inverse DFT on the measured interferograms to obtain a set of A-scans, the A-scans may be processed to unwrap the optical phase, and the expression above, or variations thereof, may be employed to extract the vibrometric measures.

$A_y$ and $\phi_y$ are both of diagnostic significance for middle ear structures and may be extracted from the measured phase data by calculating the cross-correlation of the measured signal with an in-phase and quadrature-phase acoustic sinusoid at the acoustic frequency or by Fourier analysis. These vibrometric measures may be represented in many forms and are generally referred to herein as a complex vibration amplitude (incorporating the magnitude and phase of the vibrometric measure). Various examples of vibrometric measures, such as displacement, velocity, acceleration, compliance, admittance and mobility may be computed from the complex optical phase shown above, as may ratios (which may be complex) of vibrometric measures taken at different spatial locations.

As described herein, vibrometry data is obtained from a Doppler OCT vibrometry system by processing multiple interferograms for each A-line and extracting the amplitude and phase behavior of the structure, in response to the acoustic stimulus, from the interferograms. A discrete Fourier transform (DFT) is obtained for each sampled interferogram (in swept-source OCT) or sampled spectrum (in spectral domain OCT), yielding, for each pixel associated with a given structure, a signal characterized by a phase that evolves as a sinusoid having the same frequency as the acoustic stimulus.

As described above, FIG. 1C provides a system 170 for performing Doppler OCT vibrometry measurements. It is noted that this system provides but one example system for performing phase-sensitive (PS) OCT measurements, and that other systems, such as a spectral domain Doppler OCT system of any configuration could be used as platform to perform the phase measurement techniques described herein. The system 170 shown in FIG. 1C is described in International Patent Application No. PCT/CA2018/051255, titled "SYSTEMS AND METHODS FOR MIDDLE EAR IMMITANCE TESTING" and filed on October 4, 2018, which is incorporated herein by reference in its entirety.

The OCT vibrometry system shown in FIG. 1C may employ a swept-source akinetic laser 12, such as the SLE-101E akinetic laser from Insight Photonics Solutions, which provides a sweep frequency of 100 kHz and a fiber-based interferometer 14. The swept source laser 1 provides a sample clock and a sweep trigger, where the sweep trigger signals the beginning of each new laser sweep (each A-scan event corresponding to an acquired interferogram). The sweep trigger may be derived from the sample clock. The sample clock and sweep trigger supply timing signals to other clocked and temporally synchronized components of the OCT vibrometry system. In some embodiments, the akinetic laser thus acts as the timing master to other system components, which themselves act as timing-slaves.

The sample clock and the sweep trigger are provided to a data acquisition card (e.g., ATS9351, 12-bit, 500 MHz PCIE digitizer, Alazar Technologies) to synchronize laser sweeping and data acquisition. The sweep trigger is also used to clock internal functions of the controller, which may include a field-programmable-gate-array that forms a component of controller 30 (e.g., MachXO2-7000HE, Lattice Semiconductor) that, through digital-to-analog converters (DAC), respectively control the scanning optics 24A and 24B and the acoustic stimulus source 20 that generates an acoustic stimulus signal for excitation of the object of interest, such as structures within the middle ear.

A digital synthesis approach may be employed to generate the scanning signals and acoustic signals. In the embodiment shown in FIG. 1C, the controller 30 is employed to send control signals to components of the system, but the synchronization of the optical beam scanning and the phase of the acoustic stimulus is determined by the sweep trigger of the laser 112.

The use of the single clock generated by the akinetic laser to synchronize the scanning mirrors 20 and the acoustic stimulus source 20 provides the advantage of synchronously controlling the phase of the acoustic stimulus during optical scanning. In the embodiment shown in FIG. 1C, a synchronized acoustic stimulus signal is generated by the FPGA (controller 30) by incrementing a counter on the rising edge of each clock cycle (i.e., on each laser sweep). This counter acts as a phase accumulator and is used to step though a lookup table containing the values of a sinusoid of a particular acoustic frequency. On each clock edge, the FPGA updates the value of a digital-to-analog converter (e.g., DAC900E, Texas Instruments) that drives an audio amplifier and speaker 20 used to excite vibrations in the sample.

Two synchronized lateral digital scanning signals (one for horizontal scanning, and one for vertical scanning) are also generated by the FPGA by incrementing a counter on the rising edge of each laser sweep clock cycle (i.e. in order to count laser sweeps). Once the desired number of laser sweeps for a particular scan configuration (image line) have completed, the scan mirror position is incremented by a chosen step size and the laser sweep counting starts over. This process is repeated once for every line in the image. Once the desired number of steps have been made (i.e. the number of lines in the image), the signal is reset to a chosen initial value and the lateral scan procedure starts over. The FPGA updates the values of two digital-to-analog converters (e.g., DAC900E, Texas Instruments) that respectively trigger mirror drivers (shown as cylinders in FIG. 1C) to drive the horizontal and vertical scanning optics / mirrors (shown as polygons in FIG. 1C, mounted to their respective driver). In some embodiments, there are two mirror drivers, each of which drive a mirror.

In the system 170 shown in FIG. 1C, the acoustic excitation remains phase-locked to the laser sweeps via the sweep trigger, and the accumulation of acoustic phase during and between consecutive image frames may be controlled and predicted deterministically. Without such phase-locking, even relatively slow-phase drifts between the acoustic stimulus and the laser sweeping would tend to average away the vibrational information. It is the synchronization of the timing components to the akinetic laser clock that allows this simple form of indexing to track acoustic phase changes and makes the processing steps well suited to parallel processing on graphical processing units (GPUs) using, for instance, the Compute Unified Device Architecture (CUDA) framework.

The system 170 shown in FIG. 1C, and the methods of signal processing described herein, are well suited when using akinetic lasers because of the characteristic way that linear sweeping over a broad bandwidth is achieved: through controlled mode-hopping in the akinetic swept-source architecture. The laser executes linear-in-frequency sweeps over a limited frequency range. When the end of that range is reached the laser undergoes a mode-hopping event. During this event the interferogram data is invalid and must be removed prior to line reconstruction. Fortunately, the mode hops are repeatable and deterministic across sweeps, such that, for a given laser calibration and set of sweep settings, the invalid data may be identified. However, the presence of these invalid points and their dependence on calibration and sweep parameters means that a system synchronized to the laser sampling clock (or the "k-clock" in the language conventionally used for swept source lasers) results in a phase error that depends on the sweep settings and calibration. In some embodiments, synchronizing to the start of the sweep with an external FPGA avoids this phase error.

Although many of the embodiments described previously are within the context of vibrometric systems and devices, it will be understood that various other configurations of a system involving a handheld integrated OCT beam delivery and imaging device may be provided, according to the present disclosure. In some embodiments, the handheld integrated OCT beam delivery and imaging device may be configured for imaging in the absence of the collection of vibrometric data. In some, the handheld integrated OCT beam delivery and imaging device that is employed during the medical procedure may be a therapeutic device, or a combined therapeutic and diagnostic device. Non-limiting examples of handheld therapeutic devices include drills, therapeutic laser, high intensity focused ultrasound transducer, radio-frequency ablation element, cryogenic element, heating element, blade, suction device, curette, scissors, bur, saw, rongeur, grasper, forceps. retractor, distractor, irrigation or injection needle and sonotrode.

Moreover, while some embodiments pertain to the use of a handheld integrated OCT beam delivery and imaging device that is configured for insertion into the body (or into an orifice of the body), other embodiments may employ a handheld integrated OCT beam delivery and imaging device, vibrometric or imaging or both, that is configured to obtain measurements of the body in a non-contact configuration.

The specific embodiments described above have been shown by way of example, and it should be understood that these embodiments may be susceptible to various modifications and alternative forms. It should be further understood that the claims are not intended to be limited to the particular forms disclosed, but rather to cover all modifications, equivalents, and alternatives falling within the spirit and scope of this disclosure.

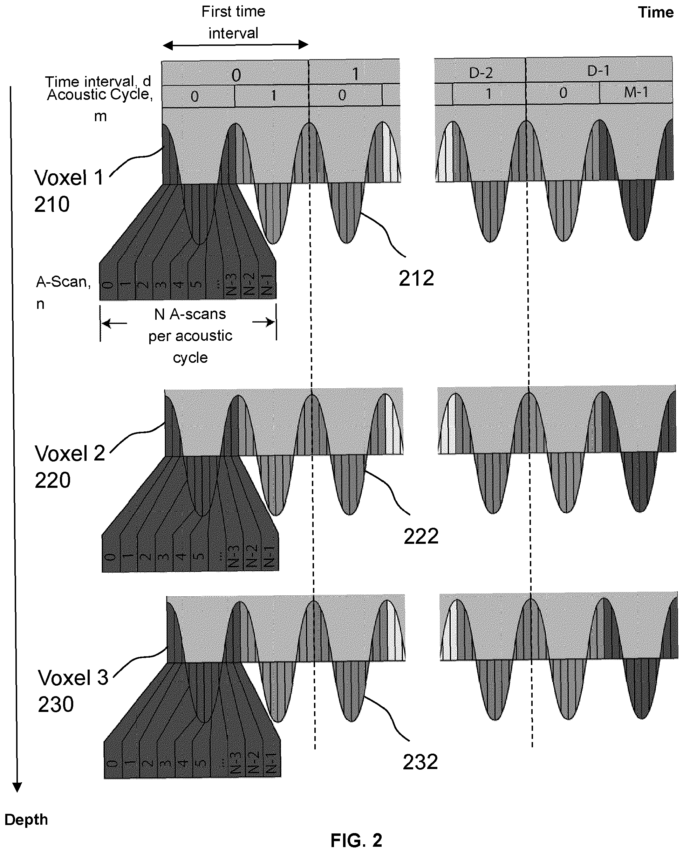

FIG. 2 depicts the spatial and temporal optical phase data, extracted from a set of interferograms acquired during the application of an acoustic stimulus using a Doppler OCT vibrometry system, according to some embodiments. FIG. 2 illustrates the depth (spatial), and time dependence of the optical phase extracted from the A-scans. The set of acquired interferograms are transformed into a corresponding set of A-scans, each A-scan having a depth dimension, and an unwrapped optical phase value is obtained for each A-scan. FIG. 2 shows the temporal phase evolution of three example voxels along the depth direction (e.g. the first three voxels among a set of voxels determined to correspond to the rigid structure) relative to the timing of the acoustic cycles. As shown in FIG. 2, each voxel, such as voxel 1 210, voxel 2 220, and voxel 3 230, has an associated optical phase time series (with one point per A-scan) that is extracted from the set of A-scans, with the optical phase evolving at the frequency of the acoustic stimulus, encoded with the vibrometric response. Although the optical phases of the three pixels, pixel 1 212, pixel 2 222, and pixel 3 232, are shown as being aligned (in phase), it will be understood these phases will generally be offset from one another due to the different depth-dependent static phase offsets associated with the imaged structure.

The present inventors recognized that it would be desirable to perform OCT imaging of the middle ear by incorporating a portion of the aforementioned optical system into a handheld OCT beam delivery device, and adapting the handheld device such that it is capable of performing both OCT imaging and generating conventional en face optical images of the exposed anatomical region. As used herein, the phrase "en face optical images" refers to non-tomographic images obtained using an imaging sensor (camera) and lens assembly that produces a digital image or video of an exposed anatomical surface.

A handheld OCT beam delivery and imaging device and associated system is described and illustrated in International Patent Application No. PCT/CA2020/05039, titled "EAR IMAGING SYSTEM" and filed on March 3, 2020, which is incorporated herein by reference in its entirety. PCT/CA2020/05039 discloses a handheld OCT beam delivery and imaging device that operates according to an optical interferometric modality for imaging and vibrometry.

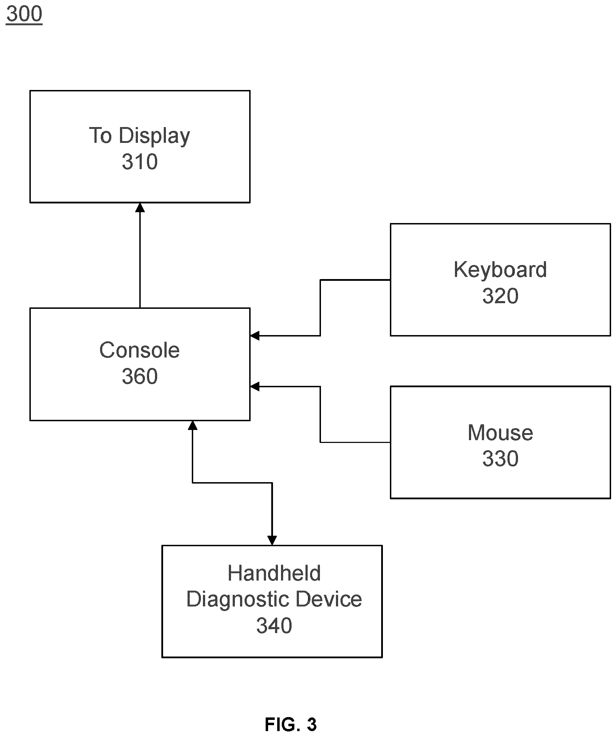

FIG. 3 illustrates a system 300 of an ear imaging system, according to some embodiments. The system 300 may be configured for the measurement of both vibrometric data and imaging data involving the ear. The handheld OCT beam delivery and imaging device 340 (handheld diagnostic device) may be configured to be held in the hand of the user and is connected via wireless or wired communication to a console 360 housing the OCT subsystem (i.e., the source, detector, and reference arm) and processing and control circuitry, and may be equipped with one or more input devices, such as a keyboard 320 and mouse 330 (and/or a foot pedal, not shown), and is interfacing to a display 310. The handheld OCT beam delivery and imaging device 340 may be connected by Bluetooth wireless technology to the control and processing console 360.

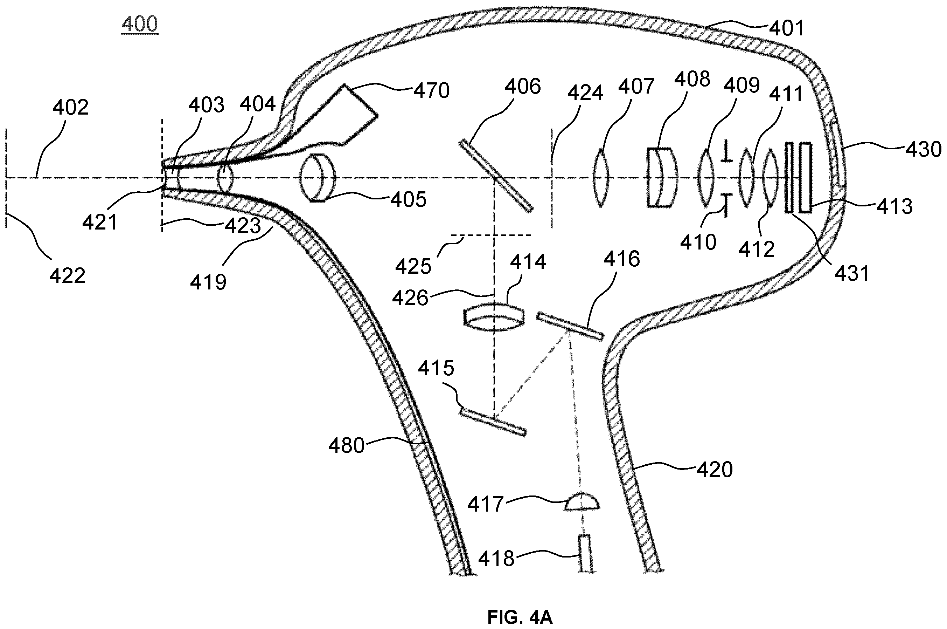

FIG. 4A depicts an illustration of the sectional view of optical system of a handheld OCT beam delivery and imaging device 400, according to some embodiments. As illustrated in FIG. 4A, the handheld OCT beam delivery and imaging device 400 may be configured to deliver the OCT beam to the inner ear, collect the scattered OCT beam, and also to collect images using an integrated imaging camera, where the OCT beam and the imaging camera share a common beam path (i.e. traverse a common set of optical elements) within the handheld device. The handheld OCT beam delivery and imaging device 400 includes an integrated beam scanning subsystem for scanning the OCT beam within a region of interest. The OCT subsystem (shown as console 360 in FIG. 3) with which the handheld OCT beam delivery and imaging device 400 is interfaced, through an optical fiber, includes an OCT light source (e.g. a laser or other low-coherence light source), the OCT reference beam path, an OCT detector, and control and processing circuitry that is described in further detail below.

The handheld OCT beam delivery and imaging device 400 is provided with a form factor similar to an otoscope, because this is a form factor that is familiar and intuitive to clinicians and may be conducive to being held very stably in the ear by a user, such as a clinician or other medical professional, who uses their fingers to brace against the patient's head. This form factor also readily allows for the incorporation of a speaker and microphone unit 470 for the delivery of an acoustic stimulus to the ear canal, which is a requirement for Doppler OCT vibrometry.

In some embodiments, the OCT beam delivery optical system 400 includes an optical bench housed within a device housing 401. The housing 401 is attached to (and may be integral with) a handle or handle portion 420, the handle or handle portion 420 is shaped so as to be comfortably held in the hand of a user (e.g., a clinician or medical professional).

Protruding from the distal front of the housing 401 is a speculum holder 419, upon which a removable speculum tip (not depicted) may be installed for disposable use in an ear of a patient. The speculum holder 419 contains embedded sound tubes to connect the microphone and speaker unit 470 integrated into the handheld device to the open cavity of the speculum and ear canal. The speculum holder 419 has a conical form with an outer diameter becoming smaller into the direction of the distal end 421 of the housing, configured to be inserted into an ear canal of a patient. Inside the housing 401 and handle 420 is an optics and electronics compartment with appropriate optics and electronics arranged therein.

Protruding from the bottom of the handle 420 may be a cable (not depicted) to connect the handheld ear imaging device 400 back to the console 360 with a computer having a display for showing images, the state of the user interface, and other clinically relevant information. A panel of indicator(s) 430 may be integrated with a handpiece indicator circuit board and may be situated upon the proximal back of the ear imaging device 400 such that the indicator(s) 430 are visible to the user; the indicator(s) 430 may provide summary information to the user, such as the current state of operation.

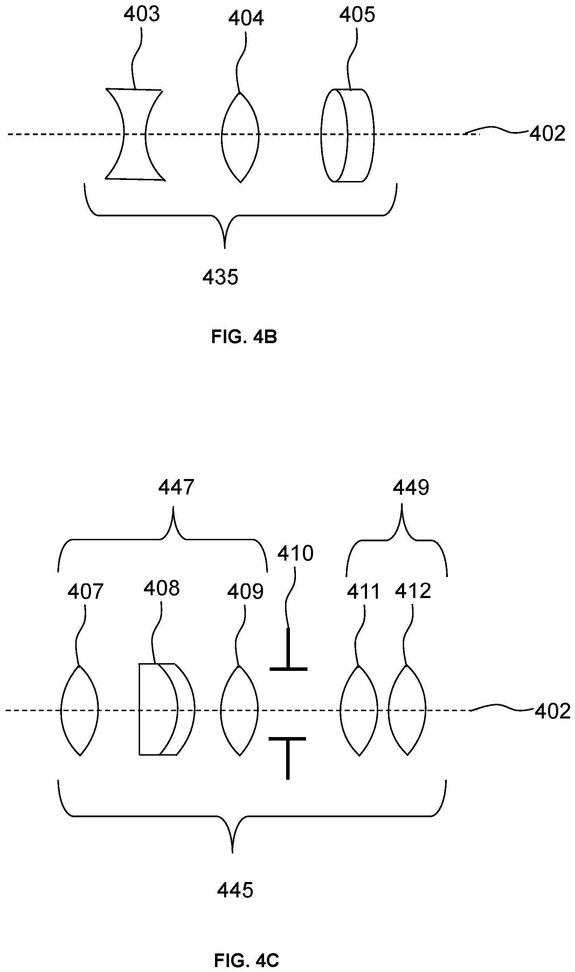

Within the housing 401 along a first optical axis 402, optical components for imaging may be arranged. These optical components may include a distal lens assembly 435, that includes lens 403, lens 404, and lens 405, depicted in FIG. 4B; an optical beam combining element 406; a proximal lens assembly 445, that includes lens 407, lens 408, lens 409, limiting aperture 410, lens 411, and lens 412, depicted in FIG. 4C; and an image sensor 413. The image sensor 413 may be a CCD sensor or the like. The optical beam combining element 406 may be arranged between the distal lens assembly 435 and the proximal lens assembly 445.

As shown in FIG. 4C, the proximal lens assembly 445 may include, in the direction of light propagation from the distal end 421 to the image sensor 413, a first lens group 447 including lens 407, lens 408, and lens 409 of positive refractive power; a second lens group 449 including lens 411 and lens 412 of positive refractive power; and an limiting aperture 410 between the first lens group 447 and the second lens group 449. Lens 409 may be a variable focal length lens, which may provide the possibility for changing the refractive power of lens 409. This variable focal length lens 409 may be arranged in the proximity of the limiting aperture 410, ideally as close as mechanically possible to the limiting aperture 410 or adjacent to the limiting aperture 410.

The first lens group 447 and the second lens group 449 may be configured to conjointly form an intermediate image of the plane of the image sensor 413 in an intermediate image plane 424, which may be between the first lens group 447 and the optical beam combining element 406. The distal lens assembly 435 may be configured to image the intermediate image plane 424 into an image plane or distal object plane 422 positioned at a predefined distance distal from the distal end 421 of the speculum holder 419.

Concurrently, the first lens group 447 and the distal lens assembly 435 may be configured to image the limiting aperture 410 into a pupil plane 423 in the proximity of the distal end 421 of the speculum holder 419. In this pupil plane 423, an entrance pupil of the optical system 400 is thereby formed by the image of the limiting aperture 410 in this pupil plane 423. Because of the small diameter of the speculum holder 419 at its distal end 421, the image of the limiting aperture 410 in the pupil plane 423 may have a reduced imaging scale. In this way, it is achieved that light rays emanating from a point in the distal object plane 422 and entering the device at its distal end 421 will converge at the image sensor 413 and will be sharply imaged onto the image sensor 413.

By changing the optical power of the variable focal length lens 409, the position of the distal object plane 422 which will be sharply imaged onto the image sensor 413 by the conjoint action of the distal lens assembly 435 and the proximal lens assembly 445 may be changed along the optical axis 402. As described herein, the variable focal length lens 409 may be positioned in the proximity of the limiting aperture 410, any change of the optical power of the variable focal length lens 409 may have no or only a very small impact on the imaging of the limiting aperture 410 into the entrance pupil plane 423 and its position along the optical axis 402.

It will be understood that the variable focal length lens 409 may be implemented according to a wide variety of components that facilitate variation in lens focal length, such as the liquid-based variable focal length lens, a mechanical lens assembly configured to vary the focal length via mechanical movement of one or more lenses of the lens assembly, a liquid crystal lens, and MEMS-based lenses. Non-limiting examples of liquid-based variable focal length lenses include electrowetting-based variable focal length lenses, electroactive polymer based variable focal length lenses, liquid crystal based variable focal length lenses, electro-mechanically shaped meniscus based variable focal length lenses, electro-mechanically shaped encapsulated-fluid based variable focal length lenses, hydraulic-based variable focal length lenses, mechanically and/or electrically actuated elastomeric lenses, and thermally-tunable variable focal length lenses. In some embodiments, the variable focal length lens 409 is configured only for varying the axial location of the focus and is not configured for use in lateral scanning of the imaging beam.

By introducing the variable focal length lens 409 into the optical bench, a user may want to look at deeper or shallower structures in an image and would have to separately adjust the focal length of the variable focal length lens to bring objects of interest into focus in the camera image and to move the OCT imaging depth region to bring the objects of interest into the OCT imaging depth region or to a particular depth location within the OCT imaging depth region (e.g. the center). Typically, users would have to separately control these two independent parameters of the system, which makes the system unintuitive and difficult to use, often resulting in a camera image focused to one depth and an OCT image centered on a different depth.

This may be solved by controlling the location of the OCT imaging depth region of the OCT subsystem that is interfaced with the handheld OCT beam delivery and imaging device and the focal length of the variable focal length lens, in response to actuation of a single, common user interface element (not pictured, e.g. a dial, slider, knob etc., either real or virtual) such that the actuation of the user interface element causes both the focal length of the variable focal length lens and the axial location of an OCT imaging depth region of the optical coherence tomography sample arm beam to move in by the same distance. It may be desirable to have the focus of the imaging system coincide with the center of the OCT imaging depth region. In this case, in response to the actuation of the user interface element controlling depth, the system is configured to simultaneously adjust the location of the OCT imaging depth region and the focal length of the variable focal length lens such that the object plane of the imaging optics is collocated with the center of the OCT imaging depth region.

The adjustment of the location of the OCT imaging depth region may be achieved, for example, by changing a length of a reference arm in the optical coherence tomography subsystem, such that the imaging depth region within the sample arm of the OCT system is moved in conjunction with the axial imaging focal zone of the imaging beam path. In an example a change of the length of the reference arm in the optical coherence tomography system may be approximately (or ideally exactly) one half of the change of the distance of the distal object plane 422 from the distal end 421 due to a change of the power of the variable focal length lens 409. To move the depth of the OCT image window by the same amount as the object plane of the en face imaging system, the optical path length in the reference arm would be changed by $\Delta x/2$ where $\Delta x$ is the amount by which the object plane is moved axially. The reference arm optical path length is increased when the en face object plane is moved distally and decreased when the object plane is moved proximally.

Arranged within the housing 401 is also the optical system for delivering the OCT beam. The end of an optical fiber 418 may form a part of the sample beam path of the OCT interferometer and being connected to the device console (not depicted) and may be arranged within the handle portion 420 of the housing 401. In addition, a coupling lens 417, and a folding mirror 416, a scanning mirror 415 (MEMS mirror), and a third lens assembly 414 are arranged within the handle portion 420 of the housing 401 in a manner that a second optical axis 426 is formed along which OCT light emitted from the end of the optical fiber is conducted in a manner that this second optical axis 426 is arranged nearly perpendicular to the first optical axis 402 and deflected by the optical beam combining element 406 into the direction of the first optical axis 402 to the distal end 421 of the speculum holder 419.

The third lens assembly 414 is configured such that together with the action of the distal lens assembly 435, an image of the scanning mirror 415 - or even more precisely the plane of the scanning axis of the scanning mirror 415 - is formed in or close to the pupil plane 423. The scanning mirror 415 serves to scan the OCT beam in one or two directions about the axis of the scanning mirror 415 to facilitate generation of sector OCT B-Scans and OCT C-Scans. By imaging the scanning mirror 415 into or close to the pupil plane 423 the OCT beam is virtually scanned in the same manner as if the scanning would happen within this pupil plane 423 at the distal end 421 of the handheld device.

Concurrently, the coupling lens 417 and the third lens assembly 414 are configured such that an intermediate image of the end face of the optical fiber 418 is formed in an OCT intermediate image plane 425. The distance between of the OCT intermediate image plane 425 and the optical beam combining element 406 along the second optical axis 426 may be the same as the distance between the intermediate image plane 424 and the optical beam combining element 406 along the first optical axis 402 so that due to the action of the distal lens assembly 435, the end face of the optical fiber 418 is imaged into a plane in object space, which may be the distal object plane 422.

In some embodiments, the variable focal lens 409 may be arranged in the proximity of the limiting aperture 410. The same result also may be achieved if the variable focal length lens 409 is arranged in a plane into which or close to which the limiting aperture 410 is imaged. However, this may require an additional relay optics to form further intermediate imaging.

Due to providing a variable focal length lens 409, restrictions accompanying a fixed optical focus of the optical imaging beam path of the imaging camera may be overcome. While a fixed focus may make the handpiece geometry suitable for a subset of human subjects, other subjects may have an ear anatomy that may result in a lack of focus of the tympanic membrane or other structures of interest on the imaging camera, such that en face images presented to the user on the user interface are out of focus. The lack of focus impairs the ability of the user to view the relevant anatomy and have confidence in the positioning of the OCT beam to interrogate specific anatomical structures of interest for vibrometric analysis.

This may be solved with the incorporation of the variable focal length lens 409 into the optical beam path of the image sensor 413. Due to the possibility to change the distance between the distal object plane 422 and the distal end 421 in FIG. 4 of the handheld device sharp imaging of the region in which the user is interested also may be realized for people with exceptional ear anatomy. Moreover, within a particular patient, the variable focal length lens allows the user to sharply focus multiple structures at different depths without moving the handpiece. This would not be possible with fixed focus optics.

In addition to the components described above, a filter 431 may be provided in the first optical axis 402 between the optical beam combining element 406 and the image sensor 413 to reduce scattered OCT light from reaching the image sensor 413. This filter 431 may be a spectral edge filter or bandpass filter being non-transmissive for the wavelength of the OCT light and transmissive for the light used for recording the en face image.

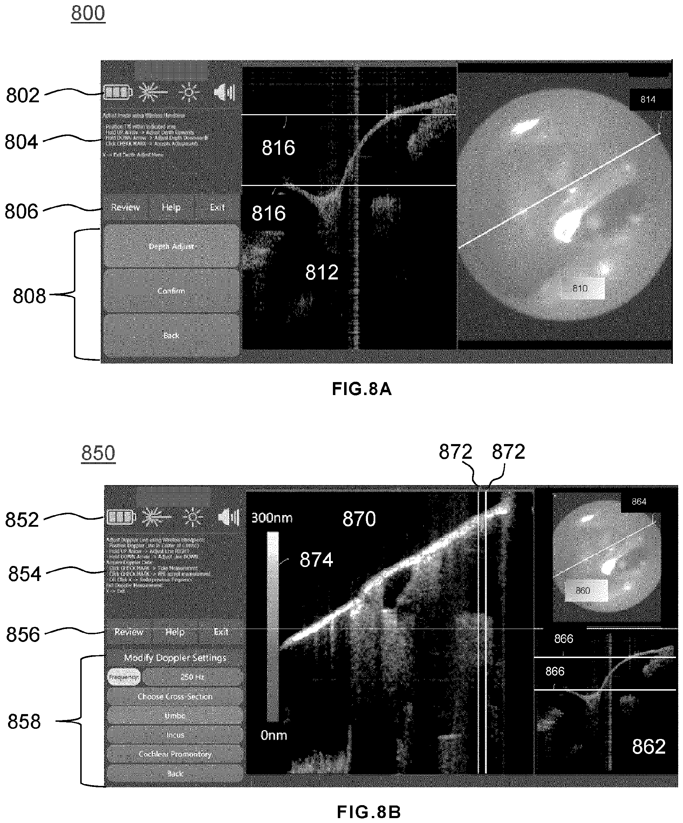

In some embodiments, the handheld OCT beam delivery and imaging device 400 may include filter 431, such as a light blocking filter. The filter 431 may be provided distal to the image sensor 413 but proximal to the optical beam combining element 406, i.e. between the image sensor 413 and the optical beam combining element 406, This light blocking filter 431 may serve to prevent even small amounts of scattered OCT light transmitted through the optical beam combining element 406. The light blocking filter 431 improves the simultaneous recording and displaying of en face images on a user interface, during acquisition of the OCT data. Such "leakage" of OCT light detected by the image sensor 413 otherwise may result in artifacts on the en face image, hampering the interpretation of such images and potentially leading to problems in understanding the actual position of the OCT beam when the en face image is annotated with OCT beam scanning annotations, as shown in FIGS. 8A and 8B. By including a filter 431 distal to the image sensor 413, the intensity of the leakage OCT light may be suppressed to a degree that is deemed satisfactory and does not result in substantial image artifacts on the en face image during OCT acquisition.

Non-limiting examples of a suitable blocking filter 431 may include a dichroic blocking filter having a blocking wavelength bandwidth configured to reflect/-transmit and/or absorb the scattered OCT light while substantially transmitting/reflecting the broadband imaging light, and/or a high-pass filter or low-pass filter configured to block the scattered OCT light while transmitting/reflecting a sufficient amount of the broadband imaging light to form an image. Another example may be one in which the imaging light and OCT light delivered to the sample are prepared with orthogonal polarizations and a polarization mode filter is used in the en face imaging arm to block the polarization of the OCT light.

While the optical bench system 400 shown in FIG. 4A relates to a beam delivery system for structural OCT, it will be understood that other components may be included to facilitate Doppler OCT vibrometry data acquisition, such as components shown in FIGS. 1C, and described herein. In some embodiments, the handheld OCT beam delivery and imaging device 400 may be configured for obtaining structural OCT data and en face optical images, Doppler OCT vibrometric data and en face optical images, combined structural OCT data and Doppler OCT vibrometric data en face optical images, or obtaining structural OCT data and/or Doppler OCT vibrometric data and en face optical images, in addition to data associated with another diagnostic modality, such as ultrasound.

In some embodiments, the overall optical system, as depicted in FIG. 4A to FIG. 4C, may include three main components: i) a distal lens assembly 435 used jointly for the OCT beam delivery system and for light used to record the en face image and which is arranged distal of an optical beam combining element 406; ii) a lens assembly coupling light to an image sensor for recording the enface image from the optical beam combining element to the image sensor; and iii) a lens and mirror assembly for providing and scanning the OCT light. One of either ii) the lens assembly coupling light to an image sensor for recoding the enface image from the optical beam combining element to the image sensor, and iii) a lens and mirror assembly for providing and scanning the OCT light may be arranged to be located mostly or entirely in the handle portion of the handheld device.

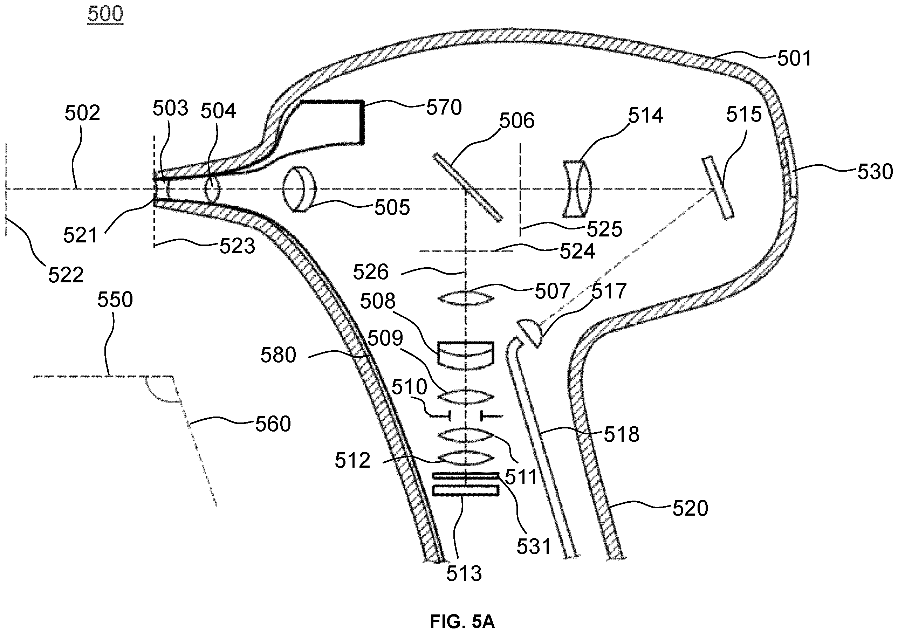

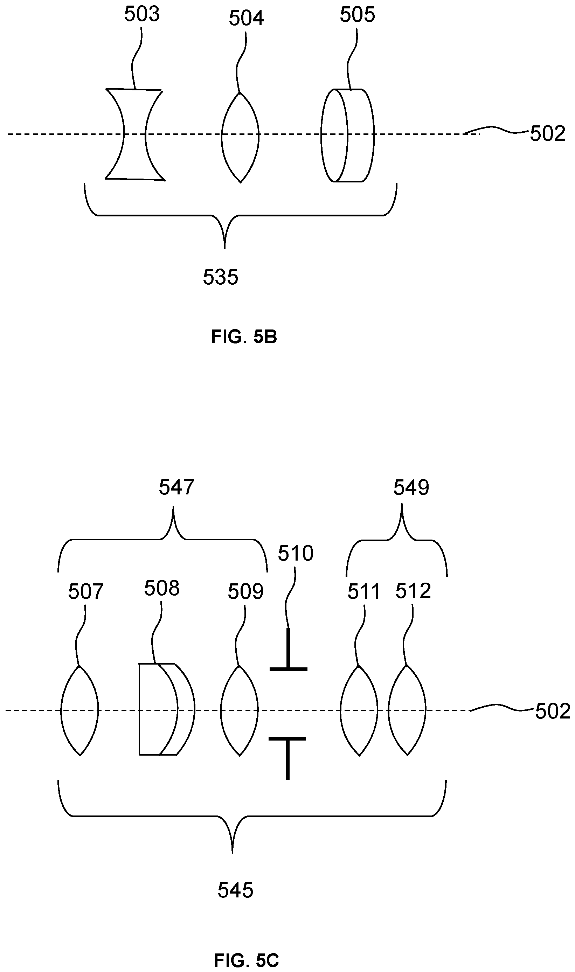

Therefore, a top-heavy configuration may be avoided which would be contrary to conventional otoscopes that have a compact and lightweight upper body portion and a weighted handle that provides the user with a sense of stability and balance. Moreover, the overall size of the handheld device may be kept compact and the center of gravity of the overall device may be kept low, to achieve a good stability in the hands of a user and providing ergonomics close to that of a traditional otoscope. Such embodiments may be depicted in FIG. 4 and FIG. 5.

In some embodiments, the optical coherence tomography beam delivery assembly OCT beam delivery assembly includes a folded optical beam path, such that an initial beam angle of the OCT sample arm beam, emerging from the optical fiber 418 within the handle portion 420 resides at an oblique angle (in FIG. 4A this angle is 90°) relative to the optical imaging axis 402, while directing the OCT sample arm beam onto the optical beam combining element 406 from a direction that is suitable for wavelength-selective reflection of the OCT beam and such that the handle portion 420 extends relative to the optical imaging axis 402, according to the oblique angle, away from the distal aperture of the device.

FIG. 5A depicts an illustration of the sectional view of optical system of a handheld OCT beam delivery and imaging device 500, according to some embodiments.

Accordingly, the handle portion may be provided such that, as indicated in the insert in FIG 5, a central axis 560 of the handle portion extends from the upper body portion (axis 550 parallel to the exit optical axis 502) along an oblique angle α that is similar to a conventional otoscope handle angle, such as, for example, at an oblique angle α, relative to the optical imaging axis 502, between 90 degrees and 135 degrees, more preferably between 100 degrees and 112 degrees, and most preferably between 105 degrees and 107 degrees.

As described herein with reference to FIG 5A to FIG. 5C, the optical bench may include an optical imaging and distal beam delivery assembly 500 for generating en face optical images. The optical imaging and distal beam delivery assembly 500 defines an optical imaging axis 502. Disposed along the optical imaging axis 502 are a distal lens assembly 535 including one more lenses (shown in FIG. 5B as lenses 503, 504, 505, and optionally including one or more additional lenses), an optical beam combining element 506, a proximal imaging lens assembly 545 (shown in FIG. 5C as lenses 507, 508, 509, 511, 512 including a variable focal length lens 509, and optionally including one or more additional lenses), and an image sensor 513, all in the handle portion 520.

Broadband light (optionally provided by an illumination source that is integrated into the handheld OCT beam delivery and imaging device may be scattered from the surface of a sample (such as a middle ear structure) residing at a distal object image plane 522 and is collected by the distal lens assembly 535, where it is reflected by the optical beam combining element 506 and is directed onto the image sensor 513 by the proximal imaging lens assembly 545. The image sensor may be any suitable 2D sensor suitable for forming an image, such as a CCD or CMOS sensor, and may have a wavelength response in the visible, infrared, or a combination thereof.

The optical beam combining element 506 may be characterized by a spectrally selective reflectivity, with a high transmissivity within the wavelength band of the OCT light, but an otherwise low broadband reflectivity, such that the broadband light scattered from the sample surface is substantially reflected by the optical beam combining element 506. An example of a suitable beam combining element 506 is a dichroic filter, and other examples include non-dichroic beamsplitters, polarizing beamsplitters, partially reflecting mirrors and fiber-based wavelength-dependent splitter/combiners and fiber-based couplers.

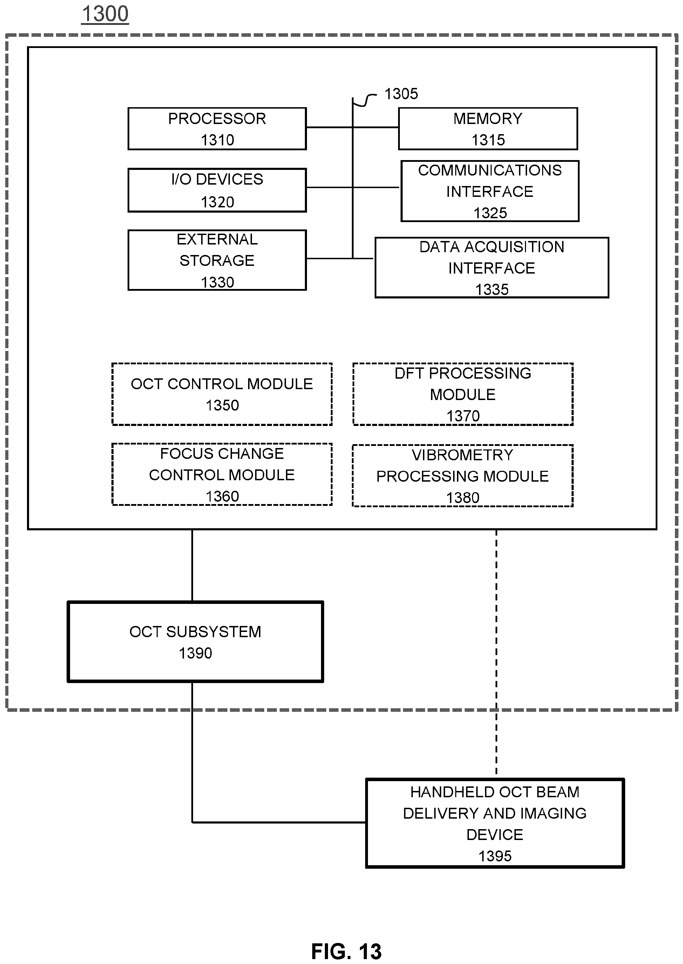

In some embodiments, a proximal optical coherence tomography beam delivery assembly configured to deliver an OCT beam, from an optical fiber 518 in optical communication with an optical coherence tomography subsystem, described herein, an optical coherence tomography sample arm beam, through a collimating lens 517, a scanning mirror 515, and optionally one additional lens 514, and onto optical beam combining element 506. The optical beam combining element 506 may be spectrally selective to be highly transmissive to the OCT light at the incident angle of the OCT light, such that the optical coherence tomography sample arm beam is directed through the distal lens assembly 535 onto a sample in the distal object plane or distal object plane 522, thereby defining an optical coherence tomography sample arm beam path. The scanning mirror 515 is controlled by a control and processing subsystem residing remote from the handheld OCT beam delivery and imaging device, further described in FIG. 13, to laterally scan the OCT beam for probing subsurface regions of the sample.

Optical coherence tomography light reflected (scattered) by the sample in the image plane 522 is collected by the distal lens assembly 535 and directed through the optical beam combining element 506 (which, as described above, may be highly transmissive to the OCT light at the incidence angle of the OCT light), through the proximal optical coherence tomography beam delivery assembly OCT beam delivery assembly , to the end face of the optical fiber 518, for delivery to the optical coherence tomography subsystem for detection, processing, and image generation.

In some embodiments, as shown in FIG. 5A, the arrangement of the optical components for recording the en face image and the OCT beam components may be interchanged, such that the OCT beam transmits the optical beam combining element 506 and the light for recording the en face image is deflected at the optical beam combining element 506. In this case, the optical combining element 506 may be highly transmissive to the OCT light but not to light used for en face imaging. In some embodiments, the OCT beam runs parallel to the optical axis 502 at the distal end 521 of the handheld device 500 between the scanning mirror 515 and the optical beam combining element 506 while the optical axis 526 for recording the en face image runs approximately perpendicular to the optical axis 502 at the distal end 521 between the optical beam combining element 506 and the image sensor 513.

The imaging principle in the embodiment in FIG. 5A to FIG. 5C is the same as in the embodiment described with reference to FIG. 4A to FIG. 4C. The variable focal length lens 509 is positioned in the proximity of the limiting aperture 510. The limiting aperture 510 is imaged by the proximal imaging lens assembly 545 into the entrance pupil plane 523 at the distal end 521 of the handheld device. And similarly, in both FIGS. 4-5, the scanning mirror 415, 515 is imaged by the combined action of the distal lens assembly 535 into or close to the distal pupil plane 423, 523.

In some embodiments, the proximal imaging lens assembly 545 includes a variable focal length lens 509 that enables the user to control the position of the focal region, i.e. the position of the distal object plane 522 along the first optical axis 502, thereby solving the aforementioned technical problem encountered by the inventors. The variable focal length lens 509 may be interfaced with a control mechanism integrated within the handheld OCT beam delivery and imaging device (such as a focus slider or a focus wheel). In some embodiments, the variable focal length lens 509 may be remotely interfaced with the control and processing circuitry described in FIG. 13 (e.g. via a wired or wireless connection) and may be controlled according to a control mechanism that is connected to the control and processing circuitry.

As shown in FIGS. 5A to 5C, the variable focal length lens 509 resides within the proximal imaging lens assembly 545, such that the variable focal length lens 509 is bypassed or not passed by the optical coherence tomography sample arm beam path. Accordingly, actuation of the variable focal length lens 509 impacts the focusing of the collected broadband light that is employed to form the en face image but does not impact the focusing of the optical coherence tomography light.

An alternative to the above-described arrangement with the variable focal length lens 509 positioned in the proximal imaging lens assembly 545 as shown in FIG. 5A to FIG. 5C is to arrange a variable focal length lens 509 in the distal imaging lens assembly 535. This would provide the advantage that actuation of the variable focal length lens affects the focusing of the optical coherence tomography sample arm beam path in the same or similar manner as the imaging for recording the en-face image.

However, since variable focal length lenses 509 require supporting electronics and cabling and typically have a lateral dimension considerably larger than the clear optical aperture of the lens. In systems with geometric constraints in the distal optical assembly, placement of the variable focal length lens 509 in the distal optics may entail reductions in usability and comfort. In the case of OCT systems used for middle ear imaging where the distal optics must pass through an otoscopic speculum and be inserted into the patient ear, expansion of the lateral dimensions of the optics may make the system incompatible with standard otoscope specula, may make the device uncomfortable to patients, or may make it difficult to position and align in the ear canal.

Furthermore, the need to pass both light used to form the en face image which is typically in the visible spectrum and OCT light which is typically in the infrared spectrum through the same variable focal length lens 509 will entail compromises on the optical performance with regard to anti-reflection coatings and choice of technologies for the variable focal length lens capable of achieving high transmittance in both spectral region used for en face imaging and the spectral region used for OCT. All these constraints are avoided by arranging the variable focal length lens in a beam portion that only passes light used for recording the en-face image, but not the OCT light.

The present inventors realized that locating the variable focal length lens 509 along the imaging beam path for recording the en-face image, but not along the OCT beam path, is advantageous to maintaining the otoscopic form factor of the device and does not result in significant adverse impact on device performance, while addressing the aforementioned need to vary the imaging focal region to accommodate spatial anatomical variations among different subjects, particularly variation in ear canal length.

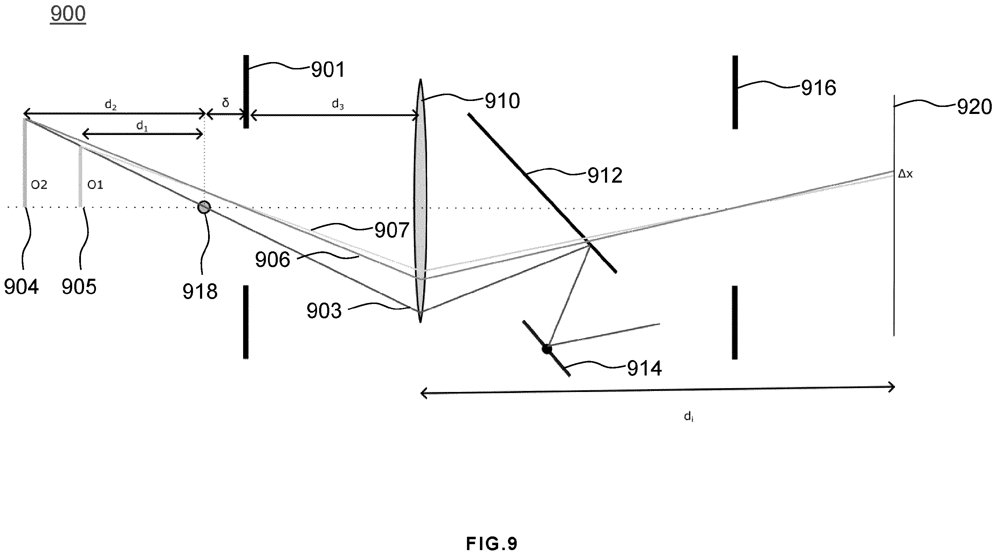

The present inventors found that incorporating the variable focal length lens 509 at a location that bypasses the OCT beam path may also lead to another technical problem that required a new technical solution. The present inventors found that it is beneficial to design the optical bench of the handheld OCT beam delivery and imaging device such that the entrance pupil plane 523 of the optical imaging beam path resides at the same axial location, along the optical imaging axis 502, as an image of the axis of the OCT scanning mirror 515 of the OCT beam path. This co-location (exact or approximate) of the entrance pupil plane 523 of the optical imaging beam path and an image of the axis of rotation of the OCT scanning mirror 515 ensures that the center of the OCT beam passes through the center of the optical imaging beam path entrance pupil regardless of the steering angle of the OCT scanning mirror or the focal length of the optical imaging system common to the OCT beam path and the imaging beam path. In other words, for each point in the object plane, the principal ray (i.e. the ray lying in the center of the ray bundle emanating from a point on the object that pass through the entrance pupil) will be coincident with the center of the OCT beam at some scanning angle over the full length of the principal ray. For points in 3D object space not lying in the object plane, the object will be out of focus in the camera image, but the circle of confusion of corresponding to the object on the image plane will be centered on the principal ray. Ray diagrams are further depicted in FIGS. 9-12.

However, the center of the "circle of confusion" for the image corresponding to the point in object space will coincide with the center of the OCT beam path for some scanning mirror angle. This implies that the center of the OCT beam will lie at the center of the image of all object points in 3D, whether they are in the object plane or outside of it. Consequently, the 3D OCT volume will be registered with the 2D camera image such that if the 2D camera image is situated in virtual 3D space at a location corresponding to the object plane of the optical imaging beam path, and the 3D OCT image is situated in virtual 3D space such that OCT image lines converge to the most distal image of the scanning mirror axis, then images in the 2D camera image arising from objects at any depth will appear centered on the 3D OCT object when viewed from the image of the center of rotation of the OCT scanning mirror.

While perfect registration (as defined herein) between the OCT volume and the 2D en face image is only achieved (in the paraxial approximation) when the entrance pupil and an image of the OCT scanning mirror axis occur at the same axial location, in practical systems some discrepancy in the location of the optical imaging beam path entrance pupil and the image of the axis of rotation of the scanning mirror may be tolerable. In some embodiments, if the axial distance along the imaging axis between the imaging beam path pupil plane and the image of the axis of the OCT scanning mirror is denoted by A, the mean distance from the center of the entrance pupil and the scanning mirror axis image to the center of the volume being imaged is D, the maximum OCT beam angle is $\theta$ and the axial extent of the image window is W, then the worst-case lateral co-registration error between the camera image and the OCT beam is $\Delta = A \frac{\theta W}{2D}$ and

it occurs at the most distal and most proximal locations in the imaging volume. Practically, it is important that this lateral registration error be kept to a small fraction (e.g. 1/10) of the size of the smallest structure intended to be imaged so that the discrepancy in the apparent location of the structure in the 2D image is not so far from the location of the 3D structure as to lead to incorrect targeting, alignment or visualization.

Maintaining close registration between images of object lying about the OCT beam path may be important for applications in which the en face image from the imaging camera is employed to determine and/or display a location or lateral spatial region associated with the acquisition of OCT data (e.g. structural OCT data and/or Doppler vibrometric OCT data). In such applications, spatial registration between the frame of reference of the en face image and the frame of reference of the scanned OCT beam is needed. In some embodiments, in the en face image, further depicted in FIG. 8, the delineation of the spatial region 814 associated with the OCT B-mode slice requires a known and fixed spatial registration between the en face image frame of reference and the OCT scanning frame of reference, even when the targeted structure does not lie in the object plane of the imaging system. For example, a user may focus the camera image such that the tympanic membrane is in focus but then target the incus (which lies distal to the tympanic membrane and will be out of focus in the camera image) for a Doppler vibrometry OCT measurement. In this case, the co-registration of the imaging entrance pupil and the distal-most image of the OCT scanning mirror axis ensures that when the OCT beam angle is selected such that the beam overlaps the center of the incus in the camera image, the OCT beam will intersect the center of the incus within 3D object space.

The present inventors discovered, through optical design simulation, that this technical problem involving the loss of spatial registration between a 2D en face image and a 3D OCT image caused by a focal-length-dependent spatial offset between the entrance pupil of the optical imaging beam path and an image of the axis of the OCT scanning mirror could be prevented or mitigated by locating the variable focal length lens at a location that is coincident or nearly coincident with the limiting aperture of the optical imaging beam path or of an image of the limiting aperture. Such an embodiment is illustrated in FIG.5 in which the limiting aperture 510 of the optical imaging beam path is adjacent to the variable focal length lens 509. The same effect could be achieved by situating the variable focal length lens at an image of the limiting aperture. This relationship between the limiting aperture 510 of the optical imaging beam path and the variable focal length lens 509 ensures that changes to focus of the en face imaging system do not cause the entrance pupil to move axially relative to the distal-most image of the axis of rotation of the OCT scanning mirror. As a result, the focus of the en face imaging system may be changed to bring different parts of the imaging volume into focus in the en face image without affecting the co-registration of the OCT image and en face image and, in particular, without introducing lateral registration errors between them.

In some embodiments, the variable focal length lens may be collocated with the limiting aperture (rather than an image of a limiting aperture), and the solution does not depend on the size of the limiting aperture, except insofar as the aperture collocated with the variable focal length mirror must be the limiting aperture for the en face imaging system. Larger limiting aperture diameters will produce a sharper focus at the expense of a shorter depth of field while smaller limiting aperture diameters will result in a weaker focus and a longer depth of field. If the desired limiting aperture size is smaller than the physical size of the variable focal length lens, then the aperture may be placed immediately distal to or proximal to the lens or the lens may be located at an image of the limiting aperture rather than at the limiting aperture itself. The limiting aperture may be placed at locations where rays emanating from the object plane are converging, at locations where they are diverging or locations where they are parallel to the optical axis.

In systems in which the rear principal plane of the variable focal length lens is offset axially from the limiting aperture, changes to the focal length of the variable focal length lens will cause the entrance pupil to move axially when the focus is changed.

In some embodiments, the spatial offset between the limiting aperture of the optical imaging beam path and an external refracting surface of the variable focal length lens 509 may be less than 4 mm, less 3 mm, less than 2 mm, or less than 1 mm. In some embodiments, the limiting aperture of the optical imaging beam path may be an optical component that is physically separate from, yet residing adjacent to, the variable focal length lens. In some embodiments, the limiting aperture of the optical imaging beam path may be directly integrated into the variable focal length lens.

In some embodiments, the limiting aperture of the optical imaging beam path may be configured and positioned relative to the variable focal length lens such that an axial offset between the entrance pupil of the optical imaging beam path and the exit pupil of the OCT beam path is less than 5 mm, less than 4 mm, less than 3 mm, less than 2 mm, or less than 1 mm when the variable focal length lens is varied throughout a prescribed focal length range. If a sufficiently small distance or special offset between the entrance pupil of the optical imaging beam path and the image of the OCT scanning mirror axis in object space is maintained in the above specified range a mis-registration between the en face images of objects lying along the OCT beam path may be maintained smaller than 1 mm, more preferably smaller than 0.5 mm and even most preferably smaller than 0.25 mm.

In some embodiments, the limiting aperture of the optical imaging beam path may be configured and positioned relative to the variable focal length lens such that criteria associated with an accuracy of spatial registration between the frame of reference of the en face image and the frame of reference of the scanned OCT beam satisfies a pre-determined criterion or criteria when the variable focal length lens is varied throughout a prescribed focal length range.

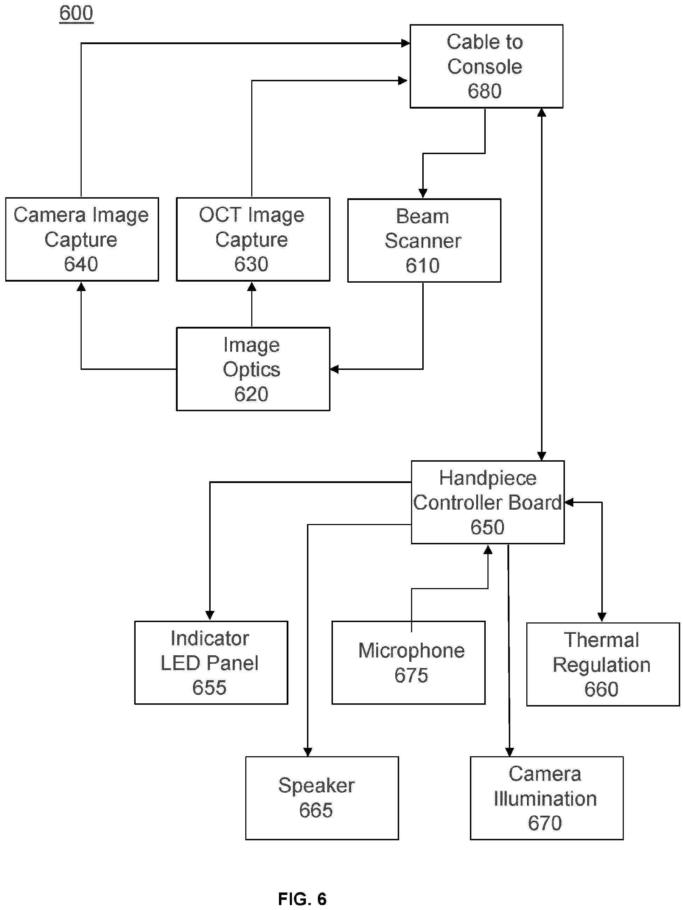

FIG. 6 illustrates a block diagram 600 of a handheld OCT beam delivery and imaging device according to some embodiments. A beam scanner 610 in the handheld device (corresponding to the scanning mirror 415 in FIG 5) may be commanded to begin scanning of the OCT beam via the console interface 680, which in some embodiments has been indirectly triggered by a controller board 650 provided in the handheld device. In some embodiments, the interface to the console 680 is wired, e.g., via fiber optic cable. The scanning OCT beam is processed through image optics 620, the output of which is captured as an OCT image at 630 and co-registered with an en face camera image at 640. These images are then relayed via the interface 680 back to the console for display, annotation, analysis, or storage. The handheld OCT beam delivery and imaging device also has a controller board 650 to support the collection of OCT and camera images. The controller board 650 is in communication with a thermal regulator 660 to keep the distal most lens of the distal lens assembly in the handheld device above the dew point in the humid environment of the ear. The board 650 also drives a light source that provides illumination 677 for camera imaging. In some embodiments an illumination source provides visible light illumination, e.g., in the wavelength range from 400 nm to 700 nm.

In some embodiments, an illumination source provides infrared light illumination, e.g., in the spectral wavelength range from 700 nm to 2000 nm, to facilitate the collection of the en face images with the imaging camera. The controller board 650 also contains controls 665 to generate some acoustic tone with a speaker and recording acoustic signals with a microphone for acoustic stimulus and sound pressure level measurement, respectively.

The device may also include one or more indicators at an indicator panel (indicator LED panel) 655, which may be indicia of controller mode, quality of image taken, status of the console, low battery of the device, or any number of other indicia as programmed. In some embodiments, the indicators are visual indicia displayed upon the handheld imaging device for easy reference by a user. In some embodiments, the indicators are one or more light emitting diodes displayed in the indicator panel. In some embodiments, the indicators may indicate sound on/off, laser on/off, illumination on/off, handpiece power on/off, error status, or combinations thereof.

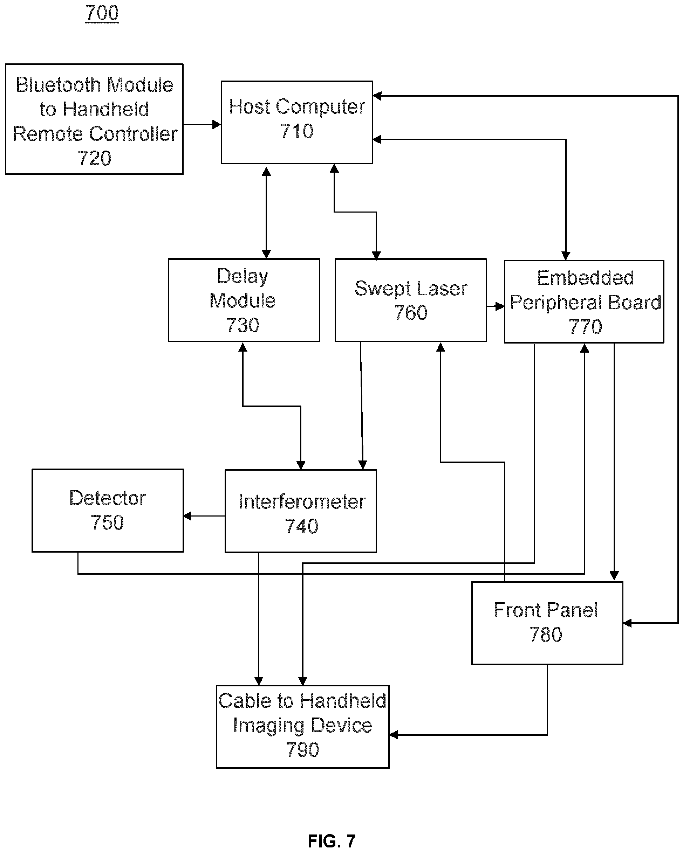

FIG. 7 illustrates a block diagram 700 of a control and processing circuitry for controlling a handheld OCT beam delivery and ear imaging device and associated OCT subsystem according to some embodiments. A host computer 710 includes one or more processors, memory, storage, and circuitry to interface with peripherals such as delay module 730, swept laser 760, peripheral board 770; to drive one or more displays for display of a graphical user interface or of indicia such as front panel 780; to perform analysis of images; and other capabilities consistent with an ear imaging system. The host computer 710 controls a swept laser 760 which feeds into an interferometer 740 which in turn feeds to a detector 750 whose output flows to the peripheral board 770. The host computer 710 is also in communication with a delay module 730 which controls the delay in one arm of the interferometer 740 to shift the image window axially. In some embodiments, user input from the front panel 780 commands the host computer 710. In some embodiments, user input from the front panel 780 directly addresses the swept laser 760 via a safety interlock driven, e.g., by the position of a key. Input from the laser 760, detector 750, interferometer 740, front panel 780, and peripheral board 770 is consolidated into the wired interface (cable 790) to the handheld imaging device.

FIG. 8A illustrates a window 800 on a computer display for an ear imaging system in brightness mode (B-mode), with the diagonal line across the en face camera image illustrating the slice selected for showing in B-mode and the upper and lower horizontal lines across the B-mode image illustrating the depth selected, according to some embodiments. A user interface window 800 for an ear imaging system in B-mode (OCT B Scan) may be displayed. The interface window 800 may display status indicators 802, user instructions 804, program menu 806, and mode command buttons 808. An en face image 810 displaying the tympanic membrane and middle ear of a subject has an overlaid diagonal line 814 across the en face image that illustrates the slice selected for showing in B-mode. The B-mode image 812 is overlaid with depth selection indicators 816 as upper and lower horizontal lines across the B-mode image 812, illustrating the depth selected for imaging in the en face image 810. The distance between the two horizontal lines may correspond to depth of focus of the en face image 810.

FIG. 8B illustrates a window on a computer display for Doppler imaging mode, with left and right vertical lines containing a region with vibration displacement amplitude information in false color, according to some embodiments. A user interface window 850 is shown for use when the handheld OCT beam delivery and imaging device 400 may be employed in Doppler vibrometry mode, displaying status indicators 852, user instructions 854, program menu 856, and mode command buttons 858. An en face image 860 displaying the tympanic membrane and middle ear of a subject has an overlaid diagonal line 864 across the en face image 860 illustrating the slice selected for showing in B-mode (OCT B Scan). The B-mode image 862 may be overlaid with depth selection indicators 866 as upper and lower horizontal lines across the B-mode image 862, illustrating the depth selected for imaging. The distance between the two horizontal lines 866 may correspond to the depth of focus of the en face image 860. At the center, a Doppler image 870 may be overlaid with Doppler selection indicators 872 as left and right vertical lines across the Doppler image 870, containing a region with vibration displacement amplitude information in false color. The false color may be interpreted by the user with assistance from the displayed color legend 874.

FIG. 9 depicts an optical system 900 for combining an OCT beam and an en face image, according to some embodiments. The system contains two objects 904 and 905 lying along an OCT beam path 903, an entrance pupil 901, objective lens 910, a beamsplitter/-combiner 912, a scanning mirror 914, a limiting aperture 916, and an image sensor 920 lying in an image plane. It should be understood that this system 900 is meant to be illustrative rather than limiting and that similar issues will appear in any system in which an optical beam combining element is used to combine an OCT beam with an en face image.

The rotation axis of the OCT scanning mirror 914 is imaged to a point in object space 918. Two objects, 904 and 905 lie along the OCT beam path 903 of the OCT system. The principal rays of these two objects 906 and 907 are those rays emanating from the objects 904 and 905 respectively that cross the optical axis at the entrance pupil of the en face imaging system. Since the entrance pupil 901 is an image of the limiting aperture 916, the plane of the entrance pupil is a conjugate plane of the limiting aperture 916, and so the principal rays 906 and 907 also cross the optical axis at the limiting aperture. In general, there may be an axial shift $\delta$ between the axial location of the image of the OCT scanning mirror axis in object space and the entrance pupil of the en face imaging system.

If $x_1$ and $x_2$ are the heights of the images of object 904 and 905 in the image plane, respectively, $\delta$ is the axial distance between the image of the OCT scanning mirror axis 918 and the entrance pupil 901 of the en face imaging system, and d1 and d2 are the axial distances from the image of the OCT scanning mirror axis in object space, then

$$\frac{\Delta x}{x_1} = \frac{\delta(d_2 - d_1)}{d_1(d_2 + \delta)}$$

From this equation, if d1 $\neq$ d2 (i.e. if the objects 904 and 905 are at different depths), then $\Delta x$ goes to zero if and only if $\delta$=0. This means that for objects lying along the OCT beam path 903 at different depths to appear at the same location in the image plane, the image of the OCT scanning mirror axis in object space must coincide with the location of the entrance pupil of the en face imaging system.

While this argument shows that in a fixed-focus en face imaging system, points along an OCT beam path 903 will appear at the same location in the image if the image of the OCT scanning mirror axis in object space coincides with the entrance pupil, a problem is created when a variable focal length lens is used to change the location of the focus. In general, changes to the focal length of a variable focal length lens lying in the proximal lens assembly 920 can change the location of either the entrance pupil 901 or the exit pupil of the en face imaging optics. In a system in which the image of the OCT scanning mirror axis in object space is initially coincident with the entrance pupil 901, a change to the focal length of the variable focal length lens may move the entrance pupil such that it is no longer coincident with the image of the OCT scanning mirror angle in object space. Alternatively, a change to the focal length of the variable focal length lens that moves the exit pupil will change the angular magnification of the en face imaging system which will cause the image of all objects lying along the same OCT beam path 903 to change their lateral distance to the optical axis in the image plane. Consequently, a mapping established between OCT beam angle and en face image location valid for one value of the focal length of the variable focal length lens will generally not be valid for any other value of the focal length.

Whether changes to the focal length of the variable focal length lens affect the location of the entrance pupil 901 or the exit pupil depends on the relative location of the limiting aperture 916 of the system and the variable focal length lens. If the limiting aperture 916 lies between the variable focal length lens and the image plane, then changes to the focal length of the variable focal length lens will move the entrance pupil 901 axially but will leave the exit pupil unchanged. Alternatively, if the variable focal length lens lies between the limiting aperture 916 and the image plane, then changes to the focal length of the variable focal length lens will change the axial location of the exit pupil but will leave the location of the entrance pupil 901 unchanged.

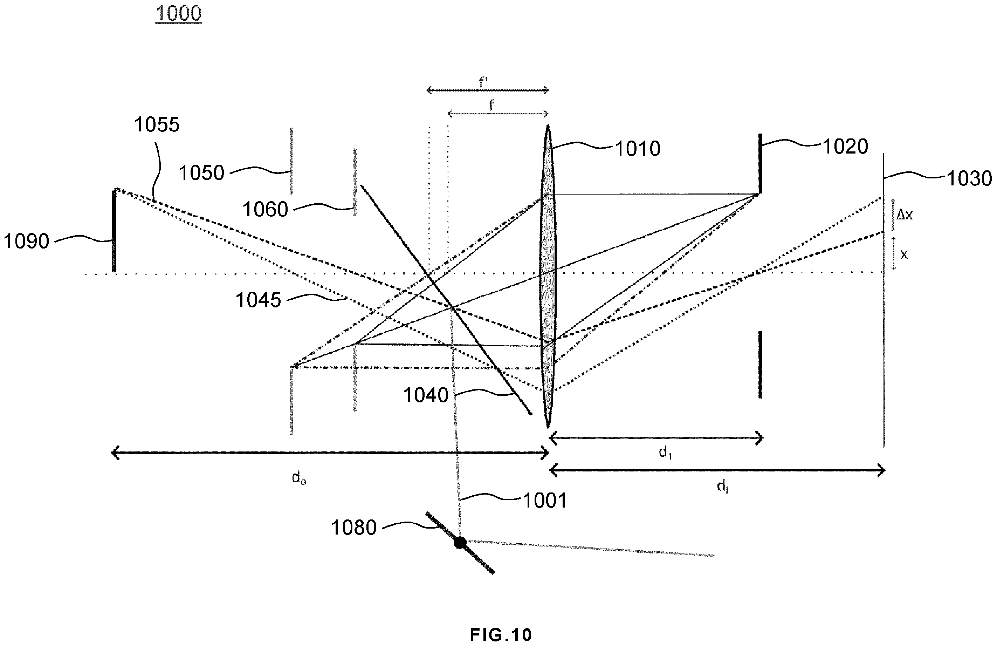

FIG. 10 shows an optical system 1000 for combining an OCT beam 1001 with an en face image in which a variable focal length lens 1010 is located distal to the limiting aperture 1020 of the en face imaging system, according to some embodiments. The optical system incorporates a beamsplitter/combiner 1040 acting as a beam combining element that combines an OCT beam 1001 with an en face image. A scanning element 1080 scans the OCT beam about an axis.

The optical system 1000 depicted in FIG. 10 is intended to be illustrative rather than limiting. The behavior of the system 1000 as described herein applies to any optical system for combining an OCT beam with an en face image in which the limiting aperture 1020 (or the image of the limiting aperture 1020 closest to the image plane 1030) lies between the principal image plane of a variable focal length lens 1010 (or the plane conjugate to it closest to the image plane) and the image plane 1030.

In FIG. 10, the axial distance between the plane of the limiting aperture 1020 and the principal image plane of the variable focal length lens 1010 is denoted as d1. In the system 1000, a change to the focal length of the variable focal length lens 1010 from f to f' causes a change in the location of the entrance pupil 1050 for focal length f to the entrance pupil 1060 for focal length f'. This movement of the entrance pupil in turn causes a change $\Delta$x in the lateral location at which the principal rays emanating from the object 1090 intersect the image plane 1030. This intersection is the center of the image of the object whether the object is in focus or out of focus. When the focal length of the variable focal length lens is f, the principal ray emanating from the object 1090 is 1045. When the focal length is f' the principal ray emanating from the object is 1055. Because of this change $\Delta$x in the center of the image, any mapping between OCT scanning angles and locations in the image made for focal length f are invalid for focal length f'.

Additionally, if the entrance pupil coincided with the image of the OCT scanning mirror axis in object space for focal length f, it will not do so for focal length f' without adjustments being made to optics in the OCT arm.

$\Delta$x is given by:

$$\frac{\Delta x}{x} = \frac{d_1 d_o \Delta f}{f(d_1 d_o - d_1 f' - d_o f')}$$

where x is height of the center of the object's image in the image plane when the variable focal length lens has a focal length f, $\Delta$x is the change in the object's height caused by the change in the focal length of the variable focal length lens from f to f', $\Delta$f=f'-f is the change in the focal length, d0 is the distance from the variable focal length lens 1010 to the object 1090, di is the distance from the variable focal length lens 1010 to the image plane 1030, and d1 is the distance from the principal image plane of the variable focal length lens 1010 to the limiting aperture 1020.

This equation applies whether the object 1090 lies in the object plane or not. In the case where the object 1090 does not lie in the object plane, the image will be out of focus but the principal ray (e.g. 1045 or 1055) will be located in the center of the circle of confusion of the out-of-focus image of the object 1090.

The equation shows that for $\Delta$x to be zero (i.e. for the image location to be unchanged by changes in the focal length of the variable focal lens 1010), then d1 must be zero, i.e. the limiting aperture 1020 (or one of its images) must be located in the principal image plane of the variable focal length lens 1010. It follows that if the principal image plane of the variable focal length lens is located in the plane of the limiting aperture 1020 (or a conjugate plane), then the location of the image of an object is independent of the focal length of the variable focal length lens 1010 when the system's limiting aperture 1020 lies between the variable focal length lens 1010 and the image plane 1030.

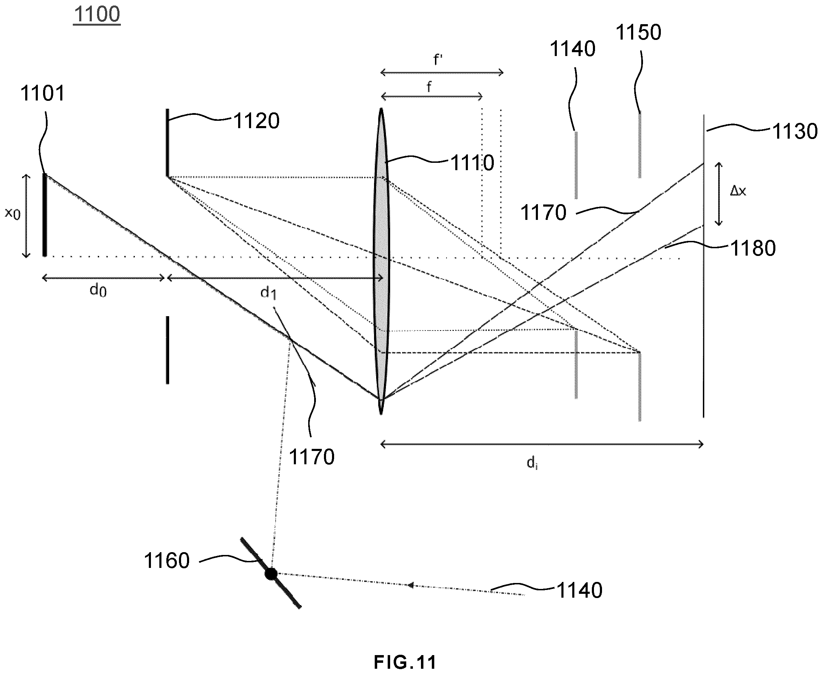

FIG. 11 shows an optical system 1100 for combining an OCT beam 1160 with an en face image on a beam combining element 1170, according to some embodiments. The system 1100 contains a variable focal length lens 1110 located between the limiting aperture 1120 of the en face imaging system and the image plane 1330. The optical system depicted in FIG. 11 is intended to be illustrative rather than limiting. The behavior of the system 1100 as described herein applies to any optical system for combining an OCT beam 1140 with an en face image in which the principal object plane of a variable focal length lens 1110 (or the plane conjugate to it closest to the image plane) is located between a limiting aperture (e.g. 1120) (or the image of a limiting aperture closest to the image plane) and an image plane (e.g. 1130).

In FIG. 11, the axial offset between the plane of the limiting aperture 1120 and the principal object plane of the variable focal length lens 1110 is denoted d1. In the system 1100, a change to the focal length from f to f' of the variable focal length lens 1110 moves and resizes the exit pupil from the location denoted 1140 when the focal length is f to the location 1150 when the focal length is f' which causes a change $\Delta$x in the lateral location of the center of the image of an object 1101. The object center in the image plane is determined by the intersection of the principal rays emanating from the object (1170 when the focal length is f and 1180 when the focal length is f'). In the absence of a remapping of the angle of the OCT scanning mirror 1160 with the locations in the image plane 1130, this effect causes a registration error $\Delta$x between the OCT image and the en face image whenever the focus is adjusted. The registration error $\Delta$x is given by:

$$\frac{\Delta x}{x_0} = -\frac{d_1 d_i \Delta f}{d_0 f f'}$$

where d1 is the axial distance from the limiting aperture to the lens, di is the distance from the lens to the image plane, $\Delta$f=f-f' is the increase in focal length, d0 is the distance from the object plane (or the nearest conjugate plane) to the limiting aperture of the system (or the nearest conjugate plane) and x0 is the height of the object.

This equation applies whether the object lies in the object plane or not. In the case where the object does not lie in the object plane, the image will be out of focus but the principal rays (e.g. 1170 and 1180) will be located in the center of the circle of confusion generated for the out-of-focus image of the object.

For non-zero $\Delta f$ and $d_i$, $\Delta x$ goes to zero if and only if di =0, i.e. if the limiting aperture (or one of its images) is located in the principal object plane of the variable focal length lens. It follows that if the principal object plane of the variable focal length lens coincides with the plane of the limiting aperture (or one of its conjugate planes), then the location of the center of the image of an object in the image plane is independent of the focal length of the variable focal length lens.

Described herein are the necessary and sufficient conditions to maintain registration between OCT scanning mirror angles and locations in the image plane of the en face imaging system across all depths and changes in the focal length of the variable focal length lens:

1) The image of the OCT scanning mirror axis in object space must coincide with the entrance pupil
2) The principal image plane of the variable focal length lens must coincide with the plane of the limiting aperture or a conjugate plane of the limiting aperture
3) The principal object plane of the variable focal length lens must coincide with the plane of the limiting aperture or a conjugate plane of the limiting aperture

For conditions 2 and 3 to simultaneously be met, the principal object plane of the variable focal lens must coincide with the principal image plane, i.e. the thin lens approximation must hold. If it does not, then the plane of the limiting aperture (or a conjugate plane) may be optimally located midway between the principal image plane and principal object plane of the variable focal length lens.

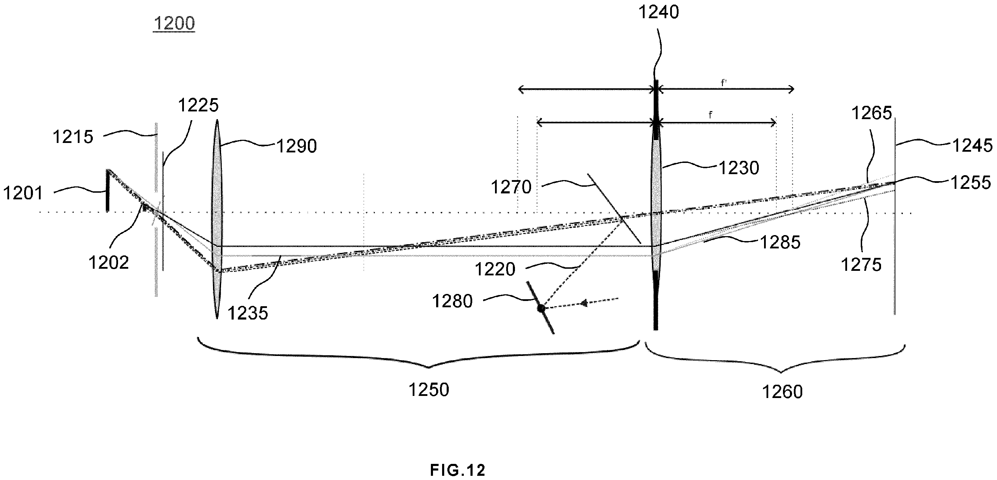

FIG. 12 shows an optical system 1200 for combining an OCT beam 1220 with an en face image, according to some embodiments. The system comprises a proximal lens assembly 1250 containing a beam combining element 1270, an OCT scanning element 1280 and an objective lens 1290, and a distal lens assembly 1260 containing a variable focal length lens 1230, a limiting aperture 1240 and an image plane 1245. The system meets all three conditions listed above in that its entrance pupil 1215 is coincident with an image plane of the axis of rotation of the OCT scanning mirror 1225 and its limiting aperture 1240 coincides with the midplane of the variable focal length lens 1230.

Having these properties, the system 1200 exhibits registration between OCT scanning element angles and lateral locations in the image that is independent of the depth of objects (e.g. 1201, 1202) or the focal length of a variable focal length lens 1230. The principal rays 1235 emanating from objects 1201 and 1202 pass through the image of the center of rotation of the OCT scanning element 1225 at the entrance pupil 1215, are refracted by the objective lens 1290, then pass through the center of the variable focal length lens 1230 at the limiting aperture 1230.

Because the principal rays 1235 pass through the center of variable focal length lens, they are not refracted, and so take the same path to the image plane 1245 regardless of the focal length of variable focal length lens 1230. When the focal length of the variable focal length lens is set to f, object 1202 is brought into focus, so that a non-principal ray emanating from 1202 intersects the principal ray at the image plane 1245. At this focal length, object 1201 is out of focus, so that while the principal ray emanating from object 1201 intersects the center of the image 1255, a non-principal ray 1265 intersects the image plane at point offset from the center of the image, forming part of the circle of confusion for the out-of-focus image. Meanwhile, non-principal rays from object 1202 such as 1295 all intersect at the same location 1255 as the principal ray since the 1202 is in focus.

When the variable focal length lens is set to a focal length of f', object 1201 is brought into focus and object 1202 is brought out of focus. For this focal length, while the principal rays 1235 from both objects intersect the image plane at the same location 1255, a non-principal ray from object 1201 intersects the image plane at a location offset from 1255, forming part of the circle of confusion for the out of focus object. Meanwhile, non-principal rays from object 1201 such as 1285 all intersect at the same location 1255 as the principal ray since the 1201 is in focus.

While conditions 1), 2), and 3) are necessary to eliminate any dependence of image location on object depth or variable focal length lens focus, in practical systems there will be an acceptable tolerance on registration error between the OCT scanning element angle and the lateral location in the image plane determined by the performance requirements for the system. The equations describing the errors $\Delta x$ for FIGS. 9-11 may be used to bound acceptable tolerances for discrepancies in the locations of the limiting aperture (or its images, including the entrance pupil), the principal planes of the variable focal length lens and the image of the axis of rotation of the OCT scanning mirror in object space.

In some embodiments, if an acceptable lateral discrepancy between the image of an object located at the proximal edge of the image window and the distal edge of the image window is 20% of the width of the proximal edge of the image window, then the maximum acceptable axial distance between the entrance pupil and the image of the axis of rotation of the OCT scanning mirror in object space is

$$\delta_{max} = \left| \frac{-0.2 d_1 d_2}{0.2 d_1 - d_1 + d_2} \right|$$

where $d_1$ and $d_2$ are the distances from the entrance pupil to the proximal edge and distal edge of the image window, respectively.

FIG. 13 illustrates a control and processing circuitry/hardware 1300 for controlling the handheld OCT beam delivery and imaging device 1095 and the OCT subsystem 1390, according to some embodiments. The control and processing hardware 1300 may include a processor 1310, a memory 1319, a system bus 1305, one or more input/output devices 1320, and a plurality of optional additional devices such as communications interface 1325, external storage 1330, and a data acquisition interface 1335. In some embodiments, a display (not shown) may be employed to provide a user interface for facilitating input to control the operation of the system 1300. The display may be directly integrated into a control and processing device (such as an embedded display) or may be provided as an external device (such as an external monitor).

The control and processing system 1300 may include or be connectable to a console that provides an interface for facilitating the user to control the handheld OCT beam delivery device 1395 and the OCT subsystem 1390. The console may include, one or more input devices, such as, but not limited to, a keypad, mouse, joystick, touchscreen, and may optionally include a display device.

The methods described herein, such as methods for controlling acquisition of OCT data (optionally Doppler OCT vibrometric data) and the processing of OCT data, and other methods described herein, may be implemented via processor 1310 and/or memory 1315, via executable instructions represented as OCT control module 1350, focus control processing module 1060, DFT processing module 1370, and vibrometric processing module 1380, respectively. Such executable instructions may be stored, for example, in the memory 1315 and/or other internal storage.

The methods described herein may be partially implemented via hardware logic in processor 1310 and partially using the instructions stored in memory 1315. Some embodiments may be implemented using processor 1310 without additional instructions stored in memory 1315. Some embodiments are implemented using the instructions stored in memory 1315 for execution by one or more microprocessors. Thus, the disclosure is not limited to a specific configuration of hardware and/or software.

It is to be understood that the system shown in FIG. 13 is not intended to be limited to the components that may be employed in a given embodiment. In some embodiments, the system 1300 may include one or more additional processors. Furthermore, one or more components of control and processing hardware 1000 may be provided as an external component that is interfaced to a processing device. Furthermore, although the bus 1005 is depicted as a single connection between all of the components, it will be appreciated that the bus 1305 may represent one or more circuits, devices or communication channels which link two or more of the components. In some embodiments, the bus 1305 may include a motherboard, and the control and processing circuity 1300 may be integrated with the OCT subsystem 1390. The control and processing hardware 1300 may include many more or less components than those shown.

Some aspects of the present disclosure may be embodied, at least in part, in software, which, when executed on a computing system, transforms an otherwise generic computing system into a specialty-purpose computing system that is capable of performing the methods disclosed herein, or variations thereof. That is, the techniques may be carried out in a computer system or other data processing system in response to its processor, such as a microprocessor, executing sequences of instructions contained in a memory, such as ROM, volatile RAM, non-volatile memory, cache, magnetic and optical disks, or a remote storage device. Further, the instructions may be downloaded into a computing device over a data network in a form of compiled and linked version. Alternatively, the logic to perform the processes as discussed above could be implemented in additional computer and/or machine-readable media, such as discrete hardware components as large-scale integrated circuits (LSI's), application-specific integrated circuits (ASIC's), or firmware such as electrically erasable programmable read-only memory (EEPROM's) and field-programmable gate arrays (FPGAs).

A computer readable storage medium may be used to store software and data which when executed by a data processing system causes the system to perform various methods. The executable software and data may be stored in various places including for example ROM, volatile RAM, nonvolatile memory and/or cache. Portions of this software and/or data may be stored in any one of these storage devices. As used herein, the phrases "computer readable material" and "computer readable storage medium" refers to all computer-readable media, except for a transitory propagating signal per se.

Various embodiments and aspects of the disclosure are described with reference to details discussed herein. The description and drawings are illustrative of the disclosure and are not to be construed as limiting the disclosure. Numerous specific details are described to provide a thorough understanding of various embodiments of the present disclosure. However, in certain instances, well-known or conventional details are not described in order to provide a concise discussion of embodiments of the present disclosure.

As used herein, the terms "comprises" and "comprising" are to be construed as being inclusive and open ended, and not exclusive. Specifically, when used in the specification and claims, the terms "comprises" and "comprising" and variations thereof mean the specified features, steps or components are included. These terms are not to be interpreted to exclude the presence of other features, steps or components.

As used herein, the term "exemplary" means "serving as an example, instance, or illustration," and should not be construed as preferred or advantageous over other configurations disclosed herein.

As used herein, the terms "about" and "approximately" are meant to cover variations that may exist in the upper and lower limits of the ranges of values, such as variations in properties, parameters, and dimensions. Unless otherwise specified, the terms "about" and "approximately" mean plus or minus 25 percent or less.

It is to be understood that unless otherwise specified, any specified range or group is as a shorthand way of referring to each and every member of a range or group individually, as well as each and every possible sub-range or sub-group encompassed therein and similarly with respect to any sub-ranges or sub-groups therein. Unless otherwise specified, the present disclosure relates to and explicitly incorporates each and every specific member and combination of sub-ranges or sub-groups.

As used herein, the term "on the order of", when used in conjunction with a quantity or parameter, refers to a range spanning approximately one tenth to ten times the stated quantity or parameter.

**Claims**

1. An optical system comprising:

a distal lens assembly defining an optical exit axis;
an optical beam combining element;
an image sensor;
an imaging lens assembly defining an imaging beam path between the optical beam combining element and the image sensor, the distal lens assembly and the imaging lens assembly configured such that imaging light scattered by a sample residing at a distal object plane is collected by the distal lens assembly and directed onto the image sensor by the imaging lens assembly according to an optical imaging beam path;
an optical coherence tomography beam delivery assembly configured to deliver, from an optical coherence tomography subsystem, an optical coherence tomography sample arm beam onto the optical beam combining element such that the optical coherence tomography sample arm beam is directed through the distal lens assembly onto the sample, thereby defining an optical coherence tomography sample arm beam path, and such that reflected optical coherence tomography light is collected by the distal lens assembly and directed to the optical coherence tomography subsystem for detection,
wherein the distal lens assembly and/or the imaging lens assembly and/or the optical coherence tomography beam delivery assembly comprises a variable focal length lens configured to variably adjust a distance between the distal lens assembly and the distal object plane along the optical exit axis, wherein a focal length of the variable focal length lens is variable within a predefined focal length range.

2. The optical system of claim 1, wherein the variable focal length lens is arranged within the imaging lens assembly.

3. The optical system of claims 1 or 2, wherein the imaging lens assembly comprises a first lens or first lens group with positive refractive power, a second lens or second lens group with positive refractive power, wherein the first lens or the first lens group is positioned between the optical beam combining element and the second lens or second lens group, and an limiting aperture arranged between the first lens or first lens group and the second lens or second lens group.

4. The optical system of claim 3, wherein the variable focal length lens is positioned in the proximity to the limiting aperture.

5. The optical system of claim 3 or 4, wherein the variable focal length lens is positioned adjacent to the limiting aperture.

6. The optical system of one of claims 3 to 5, wherein the first lens or first lens group and the distal lens assembly are configured to generate an image of the limiting aperture in proximity to a distal end of the distal lens assembly, the image of the limiting aperture thereby defining an entrance pupil of the optical imaging beam path leading from the distal object plane to the image sensor.

7. The optical system of one of claims 1 to 6, wherein the optical coherence tomography beam delivery assembly comprises a scanning mirror having a scanning mirror axis and one or more than one additional lens configured to conjointly with the distal lens assembly generate an image of the scanning mirror axis, the image of the scanning mirror axis being in proximity to the entrance pupil of the optical imaging beam path.

8. The optical system of one of claims 3 to 7, wherein the first lens or first lens group, the one or more additional lenses, the distal lens assembly and a positioning of the variable focal length lens are configured such that a distance between an entrance pupil associated with an optical imaging beam path leading from the distal object plane to the image sensor and the image of the scanning mirror axis associated with the optical coherence tomography sample arm beam path remains less than 4 mm when the variable focal length lens is varied throughout the predefined focal length range.

9. The optical system of one of claims 3 to 8, wherein the first lens or lens group, the one of more additional lenses, the distal lens assembly and a positioning of the variable focal length lens are configured such that a mis-registration between images recorded with the image sensor of objects laying along a same OCT path remains smaller than 1 mm when the variable focal length lens is varied throughout the predefined focal length range.

10. The optical system according to any one of claims 1 to 9, wherein the variable focal length lens is a liquid lens.

11. The optical system according to any one of claims 1 to 10, wherein the optical coherence tomography subsystem is configured such that a change in an axial location of the distal object plane caused by actuation of the variable focal length lens is accompanied with a corresponding change in an axial location of an imaging depth region of the optical coherence tomography sample arm beam.

12. The optical system according to claim 11, wherein the axial location of the axial focal region of the optical coherence tomography sample arm beam is varied by changing a length of a reference arm of the optical coherence tomography subsystem.

13. An optical system comprising:

a distal lens assembly defining an optical exit axis;
an optical beam combining element;
an image sensor;
an imaging lens assembly defining an imaging beam path between the optical beam combining element and the image sensor, the distal lens assembly and the imaging lens assembly configured such that imaging light scattered by a sample residing at a distal object plane is collected by the distal lens assembly and is directed onto the image sensor by the imaging lens assembly according to an optical imaging beam path;
an optical coherence tomography beam delivery assembly configured to deliver, from an optical coherence tomography subsystem, an optical coherence tomography sample arm beam onto the optical beam combining element such that the optical coherence tomography sample arm beam is directed through the distal lens assembly onto the sample, thereby defining an optical coherence tomography sample arm beam path, and such that reflected optical coherence tomography light is collected by the distal lens assembly and directed to the optical coherence tomography subsystem for detection; the optical system further comprising an optical filter, located within the imaging beam path, wherein the optical filter is configured to attenuate scattered laser light of the optical coherence tomography subsystem.

14. The optical system according to one of claims 1 to 13, wherein the optical system is integrated into a handheld device.

15. A system comprising an optical system according to one of claims 1 to 14, a control and a control interface, wherein the control is configured to adjust a length of a reference arm of the optical coherence tomography subsystem and a focal length of a variable focal length lens due to a user input to the control interface.

16. The system of claim 15, wherein the control is configured such that a change adjustment in the length of the reference arm of the optical coherence tomography subsystem is approximately one half a change in a distance of the object plane achieved through adjustment of the focal length of the variable focal length lens.

17. The system of claims 15 or 16, wherein the optical coherence tomography subsystem is an optical coherence tomography vibrometry subsystem comprising a speaker and a microphone integrated into a handpiece.

18. The system of one of claims 15 to 17, wherein the optical coherence tomography subsystem is one of a swept source optical coherence tomography subsystem and a spectral domain optical coherence tomography subsystem.

19. A handheld optical device comprising:

a housing comprising a body portion and a handle portion extending from the body portion;
an optical system comprising:

a distal lens assembly defining an optical exit axis;

an optical beam combining element;

an image sensor;

an imaging lens assembly defining an imaging beam path between the optical beam combining element and the image sensor, the distal lens assembly and the imaging lens assembly configured such that imaging light scattered by a sample residing at a distal object plane is collected by the distal lens assembly and is directed onto the image sensor by the imaging lens assembly according to an optical imaging beam path;

an optical coherence tomography beam delivery assembly configured to deliver, from an optical coherence tomography subsystem, an optical coherence tomography sample arm beam onto the optical beam combining element such that the optical coherence tomography sample arm beam is directed through the distal lens assembly onto the sample, thereby defining an optical coherence tomography sample arm beam path, and such that reflected optical coherence tomography light is collected by the distal lens assembly to the optical coherence tomography subsystem for detection;

the distal lens assembly and the optical beam combining element residing within the body portion, and

at least one of the optical imaging lens assembly and the optical coherence tomography beam delivery assembly residing at least partly in the handle portion.

20. The handheld device of claim 19, wherein the optical coherence tomography beam delivery assembly comprises a folding mirror in the beam path between an end face of an optical fiber and a scanning mirror.

21. The handheld device of claims 19 or 20, wherein the handle portion extends in a direction including an oblique angle relative to the optical exit axis in a range between 95 degrees and 120 degrees.

100

FIG. 1A

150

112

110

115

Light
Source

Fiber
Coupler

Stationary
Reference
Mirror

Grating

150

Line
Camera

Transverse
scan

120

142

155

Spectral Interferogram

Fourier
Transform

Axial scan

145

FIG. 1B

170

FIG. 1C

FIG. 2

300

```
┌─────────────────┐
│   To Display    │
│      310        │
└─────────────────┘
         ▲
         │
┌─────────────────┐        ┌─────────────────┐
│                 │◄───────│    Keyboard     │
│    Console      │        │      320        │
│      360        │        └─────────────────┘
│                 │◄───────┐
└─────────────────┘        │┌─────────────────┐
         ▲                  │    Mouse        │
         │                  │     330         │
         │                 └─────────────────┘
┌─────────────────┐
│    Handheld     │
│ Diagnostic Device│
│      340        │
└─────────────────┘
```

**FIG. 3**

FIG. 4A

403    404    405

435

**FIG. 4B**

447    449

410

407    408    409    411    412

402

445

**FIG. 4C**

FIG. 5A

FIG. 5B

FIG. 5C

FIG. 6

700

**FIG. 7**

800

FIG.8A

850

FIG.8B

**FIG.9**

EP 4 710 841 A1

**FIG.10**

FIG.11

EP 4 710 841 A1

FIG.12

1300

1305

PROCESSOR
1310

MEMORY
1315

I/O DEVICES
1320

COMMUNICATIONS
INTERFACE
1325

EXTERNAL
STORAGE
1330

DATA ACQUISITION
INTERFACE
1335

OCT CONTROL MODULE
1350

DFT PROCESSING
MODULE
1370

FOCUS CHANGE
CONTROL MODULE
1360

VIBROMETRY
PROCESSING MODULE
1380

OCT SUBSYSTEM
1390

HANDHELD OCT BEAM
DELIVERY AND IMAGING
DEVICE
1395

FIG. 13

# EP 4 710 841 A1

Europäisches
Patentamt
European
Patent Office
Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 24 20 0913

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/355139 A1 (DE VRIES HAAIJE RIMMER [NL]) 9 November 2023 (2023-11-09) <br> * paragraph [0005] - paragraph [0007] * <br> * paragraph [0012] * <br> * paragraph [0037] * <br> * paragraph [0055] * <br> * paragraph [0127] - paragraph [0128] * <br> * paragraph [0139] - paragraph [0143] * <br> * paragraph [0151] - paragraph [0154] * <br> ----- | 1-12, 14-18 | INV. <br> A61B5/00 <br> A61B3/10 <br> G01B9/02055 <br> G01B9/02091 |
| X | US 2017/245755 A1 (HÖGELE ARTUR [DE] ET AL) 31 August 2017 (2017-08-31) <br> * paragraph [0028] * <br> * paragraph [0052] * <br> * paragraph [0066] * <br> * paragraph [0085] * <br> * paragraph [0096] * <br> * paragraph [0127] * <br> * paragraph [0148] - paragraph [0149] * <br> * paragraph [0155] * <br> * paragraph [0160] - paragraph [0164] * <br> ----- | 1-12, 14-18 | |

|  | TECHNICAL FIELDS SEARCHED (IPC) |
|---|---|
|  | A61B <br> G01B |

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 29 April 2025 | Gooding Arango, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04E07)

42

Europäisches
Patentamt

European
Patent Office

Office européen
des brevets

**INCOMPLETE SEARCH
SHEET C**

**Claim(s) completely searchable:**
        1-12

**Claim(s) searched incompletely:**
        14-18

**Claim(s) not searched:**
        13, 19-21

**Reason for the limitation of the search:**

The search has been restricted to the subject-matter indicated by the applicant in their letter of 09-04-2025 filed in reply to the invitation pursuant to Rule 62a(1) and/or Rule 63(1) EPC.

## ANNEX TO THE EUROPEAN SEARCH REPORT
## ON EUROPEAN PATENT APPLICATION NO.

EP 24 20 0913

29-04-2025

| Patent document cited in search report | Publication date | Patent family member(s) | Publication date |
|---|---|---|---|
| US 2023355139 A1 | 09-11-2023 | EP 4185189 A1 | 31-05-2023 |
| | | NL 2026138 B1 | 28-03-2022 |
| | | US 2023355139 A1 | 09-11-2023 |
| | | WO 2022018285 A1 | 27-01-2022 |
| US 2017245755 A1 | 31-08-2017 | CH 711778 B1 | 14-06-2019 |
| | | DE 102015012387 A1 | 24-03-2016 |
| | | DE 112015004256 A5 | 01-06-2017 |
| | | EP 2997880 A1 | 23-03-2016 |
| | | JP 6696977 B2 | 20-05-2020 |
| | | JP 2017533004 A | 09-11-2017 |
| | | US 2016081545 A1 | 24-03-2016 |
| | | US 2017245755 A1 | 31-08-2017 |
| | | WO 2016041640 A1 | 24-03-2016 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5363839 A
- US 5919130 A
- US 8115934 B
- US 20170251924 A

- CA 2016051199 W
- CA 2018051255 W
- CA 202005039 W

**Non-patent literature cited in the description**

- **PITRIS, C. et al.** High-resolution imaging of the middle ear with optical coherence tomography: A feasibility study. *Arch Otolaryngol Head Neck Surg.*, 2001, vol. 127, 637-642